(19) **Europäisches Patentamt / European Patent Office / Office européen des brevets**

(11) **EP 2 019 148 A1**

(12) **EUROPEAN PATENT APPLICATION**

(43) Date of publication:
**28.01.2009 Bulletin 2009/05**

(51) Int Cl.:
**C12Q 1/68** (2006.01)

(21) Application number: **08006655.8**

(22) Date of filing: **24.09.2003**

| | |
|---|---|
| (84) Designated Contracting States:<br>**AT BE BG CH CY CZ DE DK EE ES FI FR GB GR HU IE IT LI LU MC NL PT RO SE SI SK TR**<br><br>(30) Priority: **28.09.2002 JP 2002327516**<br>**09.12.2002 JP 2002383869**<br><br>(62) Document number(s) of the earlier application(s) in accordance with Art. 76 EPC:<br>**03103543.9 / 1 405 923**<br><br>(27) Previously filed application:<br>**24.09.2003 EP 03103543**<br><br>(71) Applicants:<br>• **Inoko, Hidetoshi**<br>**Yokohama-shi,**<br>**Kanagawa-ken 245-0002 (JP)**<br>• **Tokai University**<br>**Shibuya-ku, Tokyo 151-8677 (JP)** | (72) Inventors:<br>• **Inoko, Hidetoshi**<br>**Yokohama-shi**<br>**Kanagawa-ken**<br>**245-0002 (JP)**<br>• **Tamiya, Gen**<br>**Tokushima University, Faculty of medicine**<br>**Tokushima-ken 770-8503 (JP)**<br><br>(74) Representative: **Turner, Craig Robert et al**<br>**A.A. Thornton & Co.**<br>**235 High Holborn**<br>**London WC1V 7LE (GB)**<br><br>Remarks:<br>•The complete document including Reference Tables and the Sequence Listing is available on CD-ROM from the European Patent Office, Vienna sub-office<br>•This application was filed on 01-04-2008 as a divisional application to the application mentioned under INID code 62. |

(54) **Gene mapping method using microsatellite genetic polymorphism markers**

(57)    A gene mapping method comprises collecting a DNA sample from to-be-screened subjects and control subjects; using forward primers having a length of 15 to 100 nucleotides and having the same nucleotide sequences as the sequence extending in 3'-direction from the 5'-terminus of each of the DNA sequences referenced with SEQ ID No: 1 to 27088 and reverse primers having a length of 15 to 100 nucleotides and having a nucleotide sequence complementary to the sequence extending in 5'-direction from the 3'-terminus of each of the DNA sequences referenced with SEQ ID No: 1 to 27088 to develop a polymerase chain reaction on the obtained DNA sample, and providing DNA sequence fragments including microsatellite genetic polymorphism markers; and statistically comparing the DNA sequence fragments obtained from the to-be-screened subjects with those obtained from the control subjects so as to identify an existing region of a pathogenic gene or a gene relating to human phenotypes with genetic factors.

**Description**

**BACKGROUND OF THE INVENTION**

**Field of the invention**

The present invention relates to methods for gene mapping using microsatellite genetic polymorphism markers. In particular, it relates to a gene mapping method, which uses a distribution map indicating the nucleotide sequences and location of each DNA sequence in a group of DNA sequences including microsatellite genetic polymorphism markers that are located on the human genome.

**Related background art**

To map pathogenic genes or genes expressing specific phenotypes, a method for comparing the genetic polymorphism markers for to-be-screened subjects and those for to-be-controlled subjects and then determining whether there is difference between the frequencies of specific alleles has been utilized.

The genetic polymorphism markers include a restriction fragment length polymorphism (RFLP), a variable number of tandem repeats (VNTR), which scatter in the human genome, microsatellites, and single nucleotide polymorphisms (SNPs); in particular, SNPs and microsatellites have attracted attention.

However, there are the following problems with usage of the SNPs as the genetic polymorphism markers. Specifically, since the SNPs are single nucleotide replacements on a genome, generally only two alleles exist, and only those SNPs existing within 5 kb to 10 kb from to-be-mapped genes correlate, genome mapping performed using the SNPs as the genetic polymorphism markers requires an enormous amount of SNPs as markers and an analysis thereof.

On the other hand, many alleles exist for a microsatellite genetic polymorphism marker and such a marker has the characteristic to show correlation even when it is positioned somewhat far from to-be-mapped genes. However, there is a problem where analyzing becomes difficult in terms of tremendous time and labor when there are too many microsatellite genetic polymorphism markers used, whereas when there are too few markers, correlation cannot be found and thus causative genes may be overlooked.

Moreover, according to the recent trend towards trying to specify a genetic mechanism that defines individual phenotypes through collection and comparison of genetic polymorphism information on a group, identifying the susceptibility gene for hereditary diseases with multiple factors or phenotypes has been started; however, the conventional methods utilizing microsatellites or SNPs can work for only limited regions and genes in the human genome, and even for the entire chromosome, only low resolution methods can be used. Therefore, there are many cases of neither being able to identify the susceptibility gene nor eliminate the possibility that other genes are involved even if that identification is successful.

**SUMMARY OF THE INVENTION**

An objective of the present invention is to provide a genomepolymorphism analyzing method, which does not have the same defects as in the gene mapping methods utilizing the SNPs or microsatellites as described above and allows clear identification of the genetic mechanism that defines individual phenotypes through collection and comparison of genetic polymorphism information on a group.

Furthermore, an objective of the present invention is to provide a protein, which is encoded by a gene that is identified by the genome polymorphism analyzing method, and antibodies against the protein.

To solve the above-mentioned problems, the present invention provides an in-vitro gene mapping method. Initially, a DNA sample may be collected from to-be-screened subjects and control subjects. The gene mapping method according to the present invention comprises the steps of: using a forward primer and a reverse primer corresponding to each DNA sequence of the DNA sequences including microsatellite genetic polymorphism markers located at intervals of 50 Kb to 150 Kb on the human genome so as to develop a polymerase chain reaction on the DNA sequence sample and obtain DNA sequence fragments including microsatellite genetic polymorphism markers; and statistically comparing the DNA sequence fragments obtained from the to-be-screened subjects' DNA with those obtained from the control subjects' DNA so as to identify an existing region for a pathogenic gene or a gene relating to human phenotypes with genetic factors.

The present invention further provides a forward primer, which has a length of 15 to 100 nucleotides and also has the same nucleotide sequences as the sequences extending in 3'-directions from the 5'-terminus of the DNA sequences including the microsatellite genetic polymorphism markers that are located on the human genome.

Furthermore, the present invention provides a reverse primer, which has a length of 15 to 100 nucleotides and also has the nucleotide sequences complementary to the sequences extending in 5'-directions from the 3'-terminus of the DNA sequences including the microsatellite genetic polymorphism markers that are located on the human genome.

Furthermore, the present invention provides a distribution map for the DNA sequences including microsatellite genetic polymorphism markers, which indicate the nucleotide sequences and location in each DNA sequence of a group of DNA sequences including microsatellite genetic polymorphism markers that are located on the human genome at intervals of 50 Kb to 150 Kb.

[Definition, abbreviation of terminology, and description of terminology]

The term "forward primer" herein denotes a primer having the same nucleotide sequence as the sequence extending in 3'-directions from the 5'-terminus of the DNA sequence including the microsatellite genetic polymorphism markers that are located on human genome.

The term "reverse primer" herein denotes a primer having a complementary nucleotide sequence to the sequence extending in 5'-directions from the 3'-terminus of the DNA sequence including the microsatellite genetic polymorphism markers that are located on human genome.

The term "distribution map" herein denotes a map indicating the distribution of DNA sequences including microsatellite genetic polymorphism markers, which are used to indicate the nucleotide sequences and location in each DNA sequence of a group of DNA sequences including microsatellite genetic polymorphism markers that are located on the human genome in desired intervals. As a group of DNA sequences including the genetic polymorphism markers, a group of DNA sequences made up of the nucleotide sequences referred to with sequence numbers 1 to 27088 are described herein.

It is noted that the nucleotide sequences with the sequence numbers 1 to 27088 are located on the human chromosome as shown below. The numbers to the right of "autosome" denote sequence numbers.

The first autosome: 1 to 2203
The second autosome: 2204 to 4561
The third autosome: 4562 to 6438
The fourth autosome: 6439 to 8005
The fifth autosome: 8006 to 9641
The sixth autosome: 9642 to 11422
The seventh autosome: 11423 to 13352
The eighth autosome: 13353 to 14629
The ninth autosome: 14630 to 15734
The tenth autosome: 15735 to 17011
The eleventh autosome: 17012 to 18202
The twelfth autosome: 18203 to 19560
The thirteenth autosome: 19561 to 20427
The fourteenth autosome: 20428 to 21178
The fifteenth autosome: 21179 to 21732
The sixteenth autosome: 21733 to 22478
The seventeenth autosome: 22479 to 23137
The eighteenth autosome: 23138 to 23801
The nineteenth autosome: 23802 to 24313
The twentieth autosome: 24314 to 24953
The twenty-first autosome: 24954 to 25307
The twenty-second autosome: 25308 to 25634
X chromosome: 25635 to 26697
Y chromosome: 26698 to 26801
Not identified locations: 26802 to 27088

The abbreviation "MS genetic polymorphism marker sequence" or "MS marker sequence" herein denotes a DNA sequence including a microsatellite genetic polymorphism marker.

The abbreviation "MS genetic polymorphism marker sequence fragment" or "MS marker sequence fragment" herein denotes a DNA sequence fragment including a microsatellite genetic polymorphism marker.

Genetic polymorphism herein indicates that two or more kinds of alleles exist on a particular gene locus with a frequency greater than 1%. A gene locus may be any region on the genome, and is not limited to the genetic region which is expressed. The term "microsatellite" denotes a sequence having di- to hexanucleotide repeats. The microsatellites are known to exist at a frequency of one in 2 kb to 3 kb on the genome. The number of repeats within each microsatellite may vary among individuals.

This variance of the number of repeats forms a polymorphism called short tandem repeat (STR). Genetic polymorphisms of microsatellites are generally determined by the number of repeats therein. A representative example of such microsatellites is the CA repeat (Dib C. et al. (1966) Nature, 380: 152-154).

For example, according to microsatellite analyses in the human HLA region made by the present inventors, microsatellites with dinucleotide repeats were found at a rate of one in approximately 8.9 kb; with trinucleotide repeats, one in approximately 12.9 kb; with tetranucleotide repeats, one in approximately 6.6 kb; with pentanucleotide repeats, one in approximately 12.6 kb; in total, one microsatellite in approximately 2.4 kb (Shiina T. et al. (1999) Proc. Natl. Acad. Sci. USA, 96: 13282-13287).

With this invention, these microsatellites are appropriately selected and used as genetic polymorphism markers. According to the present invention, a microsatellite for the genome is selected, and a distribution map for DNA sequences including microsatellite genetic polymorphism markers, which indicate the nucleotide sequence and location in each DNA sequence of a group of DNA sequences including microsatellite genetic polymorphism markers located on the human genome in intervals of 50 to 150 Kb, preferably 80 to 120 Kb, more preferably 90 to 110 Kb, is made and utilized.

The inventors made a distribution map where a group of DNA sequences including microsatellite genetic polymorphism markers on the human genome are a group of DNA sequences made up of nucleotide sequences referenced with sequence numbers 1 to 27088. The sequence table with the sequence numbers shows chromosome numbers for the respective DNA sequences, locations on the chromosomes, and distances from before and after each DNA sequence.

It is noted that "additional information" (<223>) in the sequence table attached herein is translated and explained. In the sequence table, a "<223> Marker ID" denotes a specific marker symbol. This specific symbol is an identification symbol attached to each microsatellite genetic marker by the applicant; it is necessary for the applicant when implementing the present invention and obtaining additional experimental data if necessary.

"<223> Located on chromosome" denotes a location on a chromosome. If it is written "<223> Located on chromosome 17", this means that a corresponding nucleotide sequence exists on the seventeenth chromosome. "<223> Distance between a terminus base of telomere on chromosomal short arm and 5'-terminus of this base sequence:" means the distance between the very end base of the telomeric short arm of a chromosome and corresponding base sequence 5'-terminus. That is, if it is written "<223> Distance between a terminus base of telomere on chromosomal short arm and 5'-terminus of this base sequence : 72160696", this means that the distance between a terminus base of the chromosomal telomeric short arm and the 5'-terminus of this base sequence is equal to 72160696 nucleotides.

"<223> Distance between 3'-terminus of neighbour sequence of sequence listing upward to telomere on chromosomal short arm and the 5'-terminus of this base sequence: 107571" means that the distance between the 3'-terminus of the neighbour sequence located on the chromosomal telomeric short arm, which is listed in the sequence table, and the 5'-terminus of this base sequence. That is, "<223> Distance between the 3'-terminus of neighbour sequence of sequence listing upward to telomere on chromosomal short arm and the 5'-terminus of this base sequence: 107571" means that the distance between the 3'-terminus of the neighbour sequence (appricon) positioned on the chromosomal telomeric short arm and the 5'-terminus of this base sequence is equal to 107571 nucleotides. It is noted that when this number is " 0 ", this means that there is no neighbour sequence.

It is further noted that the MS genetic polymorphism markers are set such that they exist at a rate of one to approximately 100 kb on the genome. This emanates from the fact that the microsatellites located within the range between 100 Kb and 200 Kb from a target gene indicate linkage disequilibrium, and that correlation with phenotypes such as diseases is found by mapping, thereby allowing identification of the target gene region. That is, since the microsatellite genetic polymorphism located 100 kb to 200 kb from the causative gene indicates linkage disequilibrium, a distribution map for MS genetic polymorphism markers located at a rate of one in approximately 100 kb is made so as to reliably detect the correlation without overlooking a causative gene and conduct most effective causative gene mapping with minimum labor.

With this invention, a rate of one in approximately 100 kb usually means an average rate of one in 50 kb to 150 kb, preferably one in 80 kb to 120 kb on average, and more preferably one in 90 kb to 110 kb on average. With this invention, the phrase "substantially located at a rate of one in approximately 100 kb" refers not only to the case where the MS genetic polymorphism markers are located at a rate of one in approximately 100 kb throughout the entire region, but also the case where some of the markers are located at a rate of one in approximately 100 kb. For example, when MS genetic polymorphism markers are located at a rate of one in approximately 100 kb in a certain region, and markers are located at a different frequency in another region, then as a whole it does not satisfy the ratio of "one in approximately 100 kb", however, as long as the MS genetic polymorphism markers are located in a certain region at a rate of one in approximately 100 kb, it is included in the definition of "substantially located at a rate of one in approximately 100 kb" according to this invention.

Incidentally, if three or more MS genetic polymorphism markers are prepared, the ratio thereof can be calculated; however, locating 5 or more is favorable to calculate that rate, more preferably 7 or more, and still more preferably 10 or more.

Moreover, to make a distribution map for MS genetic polymorphism markers, which are used for gene mapping, the MS genetic polymorphism markers to be prepared should have high information content available for analysis. The larger the allele number, for example, or the higher the heterozygosity, the higher information content available for the analyses.

The "allele number" indicates the number of alleles of a certain gene, according to the present invention. That is, genomic sequences at a specific gene locus having different nucleotide sequences are referred to as having an "allelic" relationship; the term corresponds to genotype, and the number thereof is referred to as the allele number. The term "average allele number" refers to the average allele number of all microsatellites used in the mapping method of this invention. Provided that the allele numbers of the 1$^{st}$ to the n-th microsatellite are denoted as $m_1$ to $m_n$, respectively, the average allele number of n microsatellites is expressed by the following equation:

$$\text{Average allele number: } (m_1 + m_2 + m_3 + \cdots + m_n)/n$$

The term "heterozygous" refers to the state of a gene in diploid organisms, such as humans, having different alleles on the two strands of chromosomes. Further, the term "heterozygosity" denotes the degree of heterozygosity. When the allele number of the x-th microsatellite is denoted as $m_x$, and the frequency of each allele $Fm_1$ to $Fm_x$, respectively, "heterozygosity ($h_x$)" of that microsatellite is given by the following equation:

$$h_x = 1 \cdot (Fm_1{}^2 + Fm_2{}^2 + Fm_3{}^2 + \ldots + Fm_x{}^2)$$

The "average heterozygosity" of n microsatellites is given by the following equation:

$$\text{Average heterozygosity: } (h_1 + h_2 + h_3 + \ldots + h_n)/n$$

Using a genetic polymorphism marker with an average allele number of 5 or more, or preferably 8 or more, and an average heterozygosity of 60% or more, preferably 65% or more, or more preferably 70% or more, more efficient mapping becomes possible.

Gene mapping according to the present invention is generally conducted by comparing the frequency of MS genetic polymorphic markers in healthy control subjects to that in randomly selected affected patients. That is, the frequency of each allele of microsatellites in healthy control subjects and the frequency of each allele of microsatellites in randomly selected affected patients are compared by correlation analysis. Herein, "randomly selected" indicates that the selected patients do not have to have a blood relation (siblings or filioparental relationship). It is preferable that the group of patients consists of patients without blood relation (siblings or filioparental relationship) to each other. If a microsatellite is within 100 kb to 200 kb from a pathogenic gene, the frequency of each allele of that microsatellite will be statistically different between healthy subjects and affected patients. A correlation analysis can be performed based on a known method (Nishimura Y. (1991) "Takei no Tokeigakuteki Riyoho" (Statistical Utilization of Polymorphisms), Saishin Igaku 46: 909-923; Oka A. et al. (1999) Hum. Mol. Genetics, 8: 2165-2170 (1999); Ota M. et al. (1999) Am. J. Hum. Genet. 64: 1406-1410 (Ozawa A. et al. (1999) Tissue Antigens 53: 263-268)).

Furthermore, not only causative genes for diseases but also those related to arbitrary phenotypes with genetic factor(s) can be mapped, for example, by randomly selecting individuals having the phenotype of interest and control individuals, and then comparing the frequency of MS genetic polymorphism markers among them.

[Target genes to be mapped]

The target genes to be mapped, according to the present invention, include all pathogenic genes and genes relating to human phenotypes with genetic factors, and are not limited to a specific range.

Diseases that have or may have genetic factors include monogenic diseases that are caused by the abnormality of a single gene, and polygenic diseases, the onset of which is triggered by the additive effect of multiple genetic factors and/or environmental factors. That is, with this invention, the term "pathogenic gene" refers not only to a single gene which can alone cause a disease, but also to a gene which is related to the onset or progress of a disease together with other genes, environmental factors, etc. Moreover, the term "pathogenic gene" herein includes genes that define the drug sensitivity of a patient to a treatment for a certain disease.

Examples of polygenic diseases include the so-called "common diseases", such as, for example, diabetes, hypertension, chronic articular rheumatism, gout, hyperlipemia, arteriosclerosis, schizophrenia, cancer, heart disease, cerebral infarction, and azoospermia, which include most of the lifestyle-related diseases. Moreover, autism, manic-depressive psychosis, epilepsy, or the like may also be included. Mapping according to the present invention allows causative gene(s) for a disease to be specified and the molecular mechanism thereof to be elucidated, and is expected to be applied to diagnosis, drug development, and development of preventive measures for the disease.

Moreover, genes relating to human phenotypes with genetic factors include causative genes relating to height, weight, skin condition, skin color, hair color, intelligence, memory, personality, and such, and the present invention may be used to map the genes. Therefore, genes expressing detectable phenotypes can be used as target genes of this invention. Furthermore, the gene mapping method of this invention can be applied not only to humans, but also to all kinds of animals including mammals, birds, and such. Psoriasis vulgaris and chronic articular rheumatism (RA), which are subjects to be mapped, according to an embodiment of the present invention is explained forthwith.

[Psoriasis vulgaris]

Psoriasis vulgaris (MIM 177900) is a common skin disorder characterized by inflammatory cell infiltration and hyperproliferation of epidermal cells. The familial nature of this disease, which affects almost 2% of Caucasian populations, has long been recognized. However, in the Japanese population, a lower incidence (0.1%) has been observed, with most psoriasis vulgaris cases being sporadic. These facts clearly suggest that psoriasis vulgaris is a multi-factorial disease triggered by the involvement of some environmental factors in individuals with a particular genetic background. In fact, through recent linkage studies on the entire genome, several susceptibility gene loci on chromosomes 6p21.3 [human leukocyte antigen (HLA)], 17q25, 4q, and many others have been identified (Tomfohrde J. et al. (1994) Science, 20, 1141- 1145; Matthews, D. et al. (1996) Nature Genet., 14, 231-233; Nair, R.P. et al. (1997) Hum. Mol. Genet., 6, 1349-1356; Trembath, R.C. et al. (1997) Hum. Mol Genet., 6,813-820).

Among them, the HLA locus is believed to be one of the major genetic factors predisposing a subject to the disease. It is well known that psoriasis vulgaris is associated with several serologically defined HLA class I antigens, such as HLA-B13, -B17, -B39, - B57, -Cw6, and -Cw7. This has been verified in many different populations throughout the world, including Caucasians and Japanese (Brenner W. et al. (1978) Arch. Dermatol. Res., 28, 337-339; Tiilikainen A. et al. (1980) Br. J. Dermatol., 102, 179-184; Ozawa A. et. al. (1981) J. Am. Acad. Dermatol., 4,205-230; Cao K. et al. (1993) Chin. Med. J., 106, 132-135; Schmitt-Egenolf, M. et al. (1996) J. Invest. Dermatol., 106, 711-714).

Among these alleles, most consistent and significant correlation is observed with HLA-Cw6. However, this correlation is not as strong as that between HLA-B27 and ankylosing spondylitis (MIM 106300) (in this case, since up to 100% of the patients carry the allele, the HLA-B-27 is likely the bona fide cause) (Moller E. and Olhagen B. (1975) Tissue Antigens, 6,237-246). In actuality, only 10% (Japanese) to 45% (Caucasians) of patients with psoriasis vulgaris carry the HLA-Cw6 allele (Tiilikainen A. et. al. (1980) Br. J. Dermatol., 102, 179-184; Asahina A. et. al. (1991) J. Invest. Dermatol., 97, 254-258). Therefore, there is a possibility that the HLA-C gene itself is not the primary locus responsible for psoriasis vulgaris and that other gene(s) located nearby harbor the true pathogenic mutation/allele with strong linkage disequilibrium to HLA-Cw6. In this respect, fine mapping of this putative susceptibility gene locus, using high resolution genetic markers around the HLA-C gene, is needed.

The present inventors have completed sequence analysis of the entire 1.8 Mb HLA class I region, from MICB (major histocompatibility complex class I chain-related gene B) to HLA-F, and have identified more than 40 new genes within this segment (Mizuki N. et al. (1997) Genomics, 42, 55-66; Shina T. et al. (1998) Genomics, 47, 372-382; Shina T. et al. (1999) Immunol. Rev., 167, 193-199). Then, for high resolution mapping of a gene presumed to be the "psoriasis gene" linked with major histocompatibility complex (MHC) in Japanese psoriasis vulgaris patients, the present inventors narrowed the target to this gene fragment, selected a total of 11 highly polymorphic novel MS genetic polymorphism markers existing at regular intervals throughout the entire 1060 kb segment around the HLA-C gene locus, and performed correlation analysis thereof. Thus, MS genetic polymorphism markers are estimated to be distributed at a density of one every 96.7 kb.

Statistical analyses of the distribution and deviation from the Hardy-Weinberg equilibrium of the allelic frequency at each microsatellite locus were carried out using these MS genetic polymorphism markers. The results revealed that the pathogenic gene for psoriasis vulgaris is located within a reduced segment of 111 kb spanning 89 kb- 200 kb of the telomeric HLA-C gene. According to RT-PCR analysis using keratinocyte mRNA, this critical region for psoriasis vulgaris included, in addition to three known genes, i.e., POU5FI (OTF3: octamer transcription factor 3) (Takeda J. et al. (1991) Nucleic Acids Res., 20, 4613-4620; Krishnan B.R. et al. (1995) Genomics, 30, 53-58), TCF19 (SC1: cell growth regulated gene) (Krishnan B.R. et al. (1995) Genomics, 30, 58-58; Ku, D.H. et al. (1991) Cell Growth Differ., 2, 179-186), and S (corneodesmosin gene) (Zhou Y. and Chaplin D.D. (1993) Proc. Natl. Acad. Sci. USA, 90, 9470-9474; Ishihara M. et al. (1996) Tissue Antigens, 48: 182-186; Tazi Ahnini, R. et al. (1999) Hum. Mol. Genet., 8,1135-1140; Allen M.H. et al. (1999) Lancet, 353, 1599-1590), four novel expressed genes identified by the genome sequencing of the entire HLC class I region, i.e., HCR (helix coiled-coil rod homologue), SPR1 (skin specific proline rich gene 1), SEEK1 (specific expressed gene in epidermal keratinocytes 1), and STG (skin specific telomeric gene) (AB029331, AB031480, AB031479, and AB031481, respectively). Accordingly, seven genes involved in sensitivity towards psoriasis vulgaris were specified.

Among them, the S gene encodes a 52 kDa - 56 kDa protein, corneodesmosin, which is expressed in differentiating epidermal keratinocytes, and thus obviously is a candidate gene related to psoriasis vulgaris. One of the four novel genes in the 11 kb critical region related to psoriasis vulgaris was expressed in most of the examined tissues, including keratinocytes, and encodes a plectin-like protein with alpha-helical coiled-coil rod domains. Plectin has been proposed to provide mechanical strength to cells and tissues by acting as a cross-linking element of the cytoskeleton (Liu C.G. et al. (1996) Proc. Natl. Acad. Sci. USA, 30, 4278-4283). Furthermore, it is of particular interest that the plectin gene is responsible for the development of epidermolysis bullosa simplex (Pulkkinen L. et al. (1996) Hum. Mol. Genet., 5, 1539-1546). The other three novel genes show no homology to any known sequences in DNA databases. However, it is noteworthy that all of the three were specifically expressed in keratinocytes and skin tissues. Thus, in addition to the S gene, these four novel genes, from their expression pattern and/or predicted function, are also promising candidate genes related to psoriasis vulgaris. As described above, the inventors have exemplified that usage of a group of DNA sequences including MS genetic polymorphism markers according to the present invention allows highly efficient gene mapping.

[Rheumatoid arthritis (RA) ]

Rrheumatoid arthritis (RA) is a progressive chronic inflammatory disease accompanied by proliferation of arthrosynovial cells and destruction of joints, cartilage, and bones, and a systemic autoimmune disease, which is as typical as systemic lupus erythematosus. The ratio of the RA patients in the entire population is approximately 0.5 to 1.0 % worldwide, and in Japan approximately 0.7 million to 1.0 million patients are estimated. There are people with a distinctly high disease rate such as Native Americans and people with an exceptionally low disease rate such as Nigerians; however, considering the difference in diagnostic criteria or the like, it is thought that the difference in disease rates of differing races is generally small.

The RA rate increases over age, but decreases after the age of sixty. In particular, the women's disease rate is two to three times the men's disease rate, and people of ages between thirty and fifty tend to catch a disease. RA onset, however, does not catenate due to sexuality. Such difference due to sexuality is considered to emanate from some resistive reaction against the RA onset occurring in men; it is pointed out that as one cause, male hormones (androgen) may be of influence. According to this, it is considered that the threshold for the accumulated risk factor that causes RA onset on women is lower than that for men.

While an understanding level for immunological aspects such as cytokine cascade has been drastically enhanced due to the progress in molecular biologic techniques for RA, the complete picture of fundamental causes of the disease has not been directly identified even though some circumstantial evidence has been identified. However, it has been insinuated based on epidemiological data collected so far that RA has a hereditary nature. The hereditary nature includes familial accumulation and disease onset match rates for monozygotic twins.

In the case of diseases with hereditary nature, the disease incidence rate for a family within which that disease is inherited is expected to be higher than that for the entire group.

Since the familial disease incidence rate is approximately 8 %, and the general-group disease incidence rate is approximately 1 %, an index representing a familial accumulation level: ?R (= familial disease incidence rate / general-group disease incidence rate) is then estimated as approximately 8. In other words, this means that the familial disease incidence rate is eight times that for the general group, insinuating that genetic factors are involved in the onset of the RA. In the case of the disease of interest not having a hereditary nature, incidence match rates (an incidence rate at which both twins suffer from the same disease) for a pair of monozygotic twins and a pair of dizygotic twins are almost the same and low; however, the stronger the hereditary nature, the higher the match rate for both types of twins, and in particular, the higher the match rate for monozygotic twins.

Aho and Simlman at, el, have reported that the incidence match rate for monozygotic twins is approximately four times that for dizygotic twins, which insinuates involvement of genetic factors. On the other hand, since the incidence match rate for the dizygotic twins is very low, it is expected that causative genetic factors or a plurality of RA susceptibility genes exist in the chromosome.

In the first step of identifying the susceptibility gene for the rheumatoid arthritis in which genetic factors are suggested to be involved, to begin with, genetic correlation analysis is carried out using a method according to the present invention so as to restrict candidate causative gene regions.

**Brief Description of the Drawings**

FIG. 1 indicates P-values obtained by the correlation test and the exact test of Hardy-Weinberg proportion with the locations of microsatellite markers used for gene mapping of psoriasis vulgaris;

FIG. 2 shows a result of carrying out the first screening of the first chromosome for rheumatoid arthritis;

FIG. 3 shows a result of carrying out the first screening of the fourth chromosome for rheumatoid arthritis;

FIG. 4 shows a profile for the rheumatism susceptibility segment in the first chromosome analyzed using a method according to the present invention;

FIG. 5 shows a profile for the rheumatism susceptibility segment in the second chromosome analyzed using a method according to the present invention;

FIG. 6 shows a profile for the rheumatism susceptibility segment in the third chromosome analyzed using a method according to the present invention;

FIG. 7 shows a profile for the rheumatism susceptibility segment in the fourth chromosome analyzed using a method according to the present invention;

FIG. 8 shows a profile for the rheumatism susceptibility segment in the fifth chromosome analyzed using a method according to the present invention;

FIG. 9 shows a profile for the rheumatism susceptibility segment in the sixth chromosome analyzed using a method according to the present invention;

FIG. 10 shows a profile for the rheumatism susceptibility segment in the seventh chromosome analyzed using a method according to the present invention;

FIG. 11 shows a profile for the rheumatism susceptibility segment in the eighth chromosome analyzed using a method according to the present invention;

FIG. 12 shows a profile for the rheumatism susceptibility segment in the ninth chromosome analyzed using a method according to the present invention;

FIG. 13 shows a profile for the rheumatism susceptibility segment in the tenth chromosome analyzed using a method according to the present invention;

FIG. 14 shows a profile for the rheumatism susceptibility segment in the eleventh chromosome analyzed using a method according to the present invention;

FIG. 15 shows a profile for the rheumatism susceptibility segment in the twelfth chromosome analyzed using a method according to the present invention;

FIG. 16 shows a profile for the rheumatism susceptibility segment in the thirteenth chromosome analyzed using a method according to the present invention;

FIG. 17 shows a profile for the rheumatism susceptibility segment in the fourteenth chromosome analyzed using a method according to the present invention;

FIG. 18 shows a profile for the rheumatism susceptibility segment in the fifteenth chromosome analyzed using a method according to the present invention;

FIG. 19 shows a profile for the rheumatism susceptibility segment in the sixteenth chromosome analyzed using a method according to the present invention;

FIG. 20 shows a profile for the rheumatism susceptibility segment in the seventeenth chromosome analyzed using a method according to the present invention;

FIG. 21 shows a profile for the rheumatism susceptibility segment in the eighteenth chromosome analyzed using a method according to the present invention;

FIG. 22 shows a profile for the rheumatism susceptibility segment in the nineteenth chromosome analyzed using a method according to the present invention;

FIG. 23 shows a profile for the rheumatism susceptibility segment in the twentieth chromosome analyzed using a method according to the present invention;

FIG. 24 shows a profile for the rheumatism susceptibility segment in the twenty-first chromosome analyzed using a method according to the present invention;

FIG. 25 shows a profile for the rheumatism susceptibility segment in the twenty-second chromosome analyzed using a method according to the present invention; and

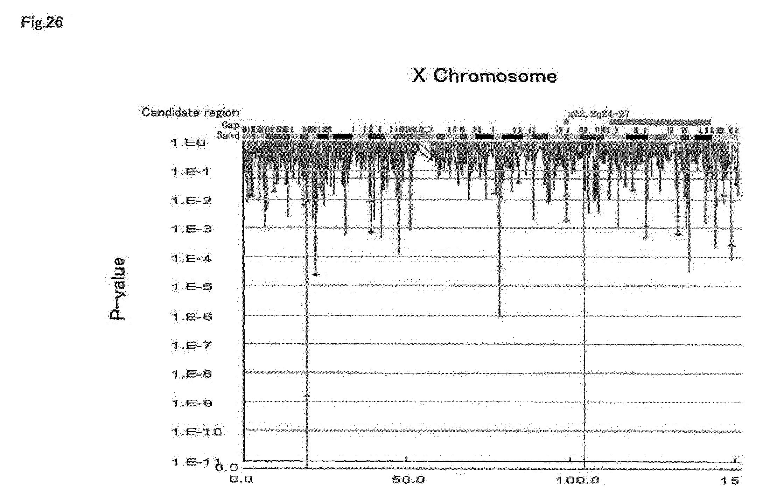

FIG. 26 shows a profile for the rheumatism susceptibility segment in the Y chromosome analyzed using a method according to the present invention.

## DESCRIPTION OF THE PREFERRED EMBODIMENTS

Identification of MS genetic polymorphisms, according to the present invention, may be carried out by: amplifying the DNA sequence samples through polymerase chain reaction (PCR) using a forward primer and a reverse primer corresponding to each DNA sequence in a group of DNA sequences including microsatellite genetic polymorphism markers located in desired intervals; performing electrophoresis using a high resolution gel such as a DNA sequencer; and performing measurement and analysis of the DNA sequence fragment including microsatellite genetic polymorphism markers, which are amplified products.

According to the present invention, before identification, a distribution map for DNA sequences including microsatellite genetic polymorphism markers, which are used to indicate the nucleotide sequence and location in each DNA sequence of a group of DNA sequences including microsatellite genetic polymorphism markers that are located in advance on the human genome at desired intervals, is made and utilized. In actuality, the inventors prepared a group of DNA sequences including microsatellite genetic polymorphism markers on the human genome, and made and utilized a distribution map including the group of DNA sequences made up of all or a part of the nucleotide sequence referenced with sequence numbers 1 to 27088. Usage of this distribution map has enabled gene mapping throughout a genome according to the present invention. It is noted that Golden Path (Dec. 22, 2001) (http://genome.ucsc.edu/) is used as the human genome reference sequence, which is utilized for making the distribution map.

The term "forward primer", according to the present invention, denotes a primer having the same nucleotide sequence as the sequence extending in 3'-direction from the 5'-terminus of the DNA sequence including the microsatellite genetic polymorphism markers that are located on the human genome, and having a length of 15 to 100 nucleotides, preferably 15 to 25 nucleotides, more preferably 18 to 22 nucleotides.

The term "reverse primer" denotes a primer having a nucleotide sequence complementary to the sequence extending in 5'-direction from the 3'-terminus of the DNA sequence including the microsatellite genetic polymorphism markers that are located on the human genome, and having a length of 15 to 100 nucleotides, preferably 15 to 25 nucleotides, more preferably 18 to 22 nucleotides.

A method according to the present invention can be easily implemented by utilizing DNA chips and mass spectrometry. Specifically, for example, by loading 1000 or more MS genetic polymorphism marker DNA sequences onto a chip, ionizing by laser irradiation, and then measuring the molecular weight using the traveled distance in a vacuum tube as an index, the number of repeats of the microsatellite, i.e., polymorphism, can be measured easily and quickly (Braun A. et al. (1997) Genomics 46: 46-23). More specifically, for example, DNA MassArray™ (MS chip) (Sequenom Co. LTD, San Diego, CA, USA; PE Biosystems Co. LTD, Foster City, CA, USA) may be used.

Moreover, through a method according to the present invention, using a forward primer and a reverse primer corresponding to the MS genetic polymorphism markers located throughout the genome, a first screening may be performed, and a second screening for the MS genetic polymorphism markers indicated positive by the first screening may then be performed. The second screening uses a different sample group, performing an analysis as with the first screening. The additional test through the second screening results in a drastic reduction of the number of the MS genetic polymorphism markers indicated false-positive without carrying out forced correction.

When the position of a target gene is restricted by mapping using the MS genetic polymorphism markers according to the present invention, candidate positions can be further restricted so as to specify the gene locus by a different mapping. For this purpose, for example, analysis using SNPs is effective. Since SNPs exist at a rate of one in 300 to 500 base pairs on the genome, with a high frequency of occurrence approaching several hundred times of that of MS genetic polymorphism markers, the SNP analysis after the mapping according to the present invention allows identification of the target gene. Specifically, after the analysis using MS genetic polymorphism markers, the polymorphism frequencies of SNPs in the candidate segments that have been considered to encompass the target gene are compared, for example, through correlation analysis between a group of patients and a group of healthy people, and such; and then, SNP markers with linkage disequilibrium detected by haplotype analysis are detected through linkage disequilibrium analysis.

To facilitate understanding of advantages of the mapping method using the MS genetic polymorphism markers according to the present invention, SNP (single nucleotide polymorphism) analysis of the human genome is explained below. The SNP analysis is a method for mapping causative genes of diseases using, as genetic polymorphism markers, 300,000 polymorphisms, which are collected based on differences due to replacements, deletions, or insertions of a single nucleotide in genes throughout the genome.

Behind the project is the idea that SNPs may be the cause of diseases with multiple factors, such diseases as the so-called lifestyle-related diseases. However, since the number of alleles of an SNP is generally only two, mapping capability is low (Kruglyak L. (1999) Nature Genetics 17: 21-24). In actuality, through analysis by the present inventors, microsatellites with 5 or more alleles that are located within approximately 200 kb from a target gene show a significant correlation, whereas the SNP analysis makes clear that only a part of SNPs that are located extremely close, within 5 kb, to the target gene showed significant correlation.

As mentioned above, this may be due to the fact that the capability in mapping SNPs is low, i.e., the number of alleles of an SNP being generally only two and its heterozygosity being 50% or less (normally 17%). Thus, according to the method of the present invention, the most effective strategy for genome mapping may be to identify a target gene by first performing mapping using a distribution map with approximately 30,000 (density of one in approximately 100 kb) MS genetic polymorphism markers located throughout the genome so as to restrict the target gene candidate segment to 100 kb, and then performing SNP analysis.

In addition, to identify a target gene from a determined sequence, for example, an exon region that may possibly be expressed can be predicted using a computer program such as GRAIL (Uberbacher E.G. and Mural R.J. (1991) Proc. Natl. Acad. Sci. USA 88: 11261-5) and GENSCAN (Burge C. and Karlin S. (1997) J. Mol. Biol. 268: 78-94), or by performing a homology search of an expressed sequence tag (EST) database for a nucleotide sequence, in which repeating sequences are removed.

Based on these results, PCR primers and probes are prepared, and intracellularly expressed fragments are then identified by RT-PCR and Northern hybridization. Furthermore, once an expressed fragment is obtained, full-length cDNA can be obtained by 5' RACE, 3' RACE, and such. Alternatively, cDNA can be isolated through screening of cDNA libraries or CapSite libraries using a fragment of the gene as a probe, and such.

Through large scale sequence analysis of the human leukocyte antigen (HLA) gene locus, along with many genes, the present inventors have identified thus far microsatellites that can be used for mapping (Mizuki N. et al. (1997) Genomics, 42, 55-66; Shiina T. et al. (1998) Genomics, 47, 372-382; Shiina, T. et al. (1999) Immunol. Rev., 167, 193-199). Using the distribution map according to the present invention, the first screening in which gene mapping is performed throughout the genome can then provide the same results. Moreover, the gene mapping methods according to this invention can be applied by restricting this HLA region.

With this invention, the term "HLA region" refers to a 3.6 Mb segment from the centromeric HSET gene to the telomeric HLA-F gene. In addition to the causative gene(s) related to psoriasis described above, those relating to other diseases are expected to be present in the HLA region.

By implementing the gene mapping method according to this invention on the HLA region, it is possible to effectively map the causative genes for such diseases. Specifically, in addition to psoriasis, examples of specific diseases for which causative genes are expected to be present in the HLA region include rheumatism, Behcet's disease, juvenile diabetes, Basedow disease, cardiomyopathy, diffuse panbronchiolitis, Buerger disease, Takayasu's disease, narcolepsy, sarcoidosis, Harada's disease, myasthenia gravis, multiple sclerosis, etc.

A causative gene for a disease specified using a mapping method according to this invention can be used for testing, preventing, and treating the disease. Genes relating to phenotypes other than diseases may also be used for tests such as genetic diagnosis and for gene therapy. Cloning identified genes can be performed by methods well known to those skilled in the art. For example, a cDNA library is made from cells in which such gene is expressed, and then prepared by performing hybridization using, as a probe, a gene fragment that is identified by mapping. The cDNA library may be prepared using, for example, the method described in the literature (Sambrook J. et al. (1989) Molecular Cloning, Cold Spring Harbor Laboratory Press), or commercially available cDNA libraries may be used instead. Alternatively, this preparation is made by: preparing RNA from cells in which the gene is expressed, synthesizing cDNA by a reverse transcriptase, synthesizing oligo-DNA based on the nucleotide sequence of the gene (or a fragment thereof), and amplifying the cDNA through PCR using the oligo-DNA as primers.

The nucleotide sequence of a full-length target gene is determined to find a translation region encoded thereby, and the amino acid sequence of the protein encoded by the gene can be obtained. Furthermore, the cDNA obtained may also be used as a probe for screening a genomic library to isolate genomic DNA.

With this invention, the term "gene" encompasses both cDNA and genomic DNA. Genomic DNA typically includes exons, introns, a promoter, and enhancers of a gene. This term also includes alleles and variants.

Cloning a target gene, which is specified using the present invention, may be carried out with the following procedure. To begin with, mRNA is isolated from a cell, tissue, or organ in which the gene is expressed. Known methods can be used to isolate mRNA; for instance, total RNA can be prepared by guanidine ultracentrifugation (Chirgwin J.M. et al. Biochemistry (1979) 18, 5294-5299) or the AGPC method (Chomezynski P. and Sacchi N., Anal. Biochem. (1987) 162: 156-159), and mRNA can be purified from total RNA using mRNA Purification Kit (Pharmacia) and such. Alternatively, mRNA may be directly prepared using QuickPrep mRNA Purification Kit (Pharmacia).

The obtained mRNA may be used to synthesize cDNA using reverse transcriptase. cDNA may be synthesized using a kit such as AMV Reverse Transcriptase First- strand cDNA Synthesis Kit (Seikagaku Kogyo). Alternatively, cDNA may be synthesized and amplified using partial sequences of a target gene as primers according to the 5'-RACE method (Frohman M.A. et al., Proc. Natl. Acad. Sci. U.S.A. (1988) 85, 8998-9002; Belyavsky A. et al., Nucleic Acids Res. (1989) 17, 2919-2932) that utilizes 5Ampli FINDER RACE Kit (Clontech) and polymerase chain reaction (PCR). A target DNA fragment is prepared from the PCR products and linked to a vector DNA. The nucleotide sequence of the target DNA can be verified by well-known methods, such as dideoxynucleotide chain termination.

The isolated DNA, as described above, is inserted into a suitable vector. When E. coli is used as the host cell, the vector is not particularly limited as long as the vector has an "ori", which is for amplifying and mass-producing the vector in E. coli (e.g., JM109, DH5a , HB101, or XL1Blue), and such, and a marker gene for selecting the transformed E. coli (e.g., a drug-resistant gene selected by a drug (e.g., ampicillin, tetracycline, kanamycin, or chloramphenicol)). For example, M13-series vectors, pUC-series vectors, pBR322, pBluescript, pCR-Script and such can be used as the vector.

Apart from the vectors, pGEM-T, pDIRECT, pT7 and such can be also used for subcloning and excision of a cDNA as well. When a vector is used to produce a protein encoded by a gene, an expression vector is especially useful.

For the purpose of expressing in E. coli, the expression vector should have the above characteristics such as being amplified in E. coli. Additionally, when E. coli, such as JM109, DH5a , HB101, or XL1-Blue, is used as the host cell, the vector should have a promoter, for example, a lacZ promoter (Ward E.S. et al. (1989) Nature 341: 544-546; Ward E.S. (1992) FASEB J.6, 2422-2427), araB promoter (Better Metal (1988) Science 240, 1041-1043), or T7 promoter, that can efficiently promote the expression of a desired gene in E.coli. Other examples of the vectors include pGEX-5X-1 (Pharmacia), "QIAexpress system" (QIAGEN), pEGFP, and pET (for this vector, BL21, a strain expressing T7 RNA polymerase, is preferably used as the host).

Furthermore, the vector may also include a signal sequence directing the secretion of the polypeptide. For producing a protein into the periplasm of E.coli, the pelB signal sequence (Lei S.P. et al., J. Bacteriol. (1987) 169, 4379) may be used as the signal sequence for protein secretion. The calcium chloride method or electroporation may be used to introduce the vector into host cells. As vectors used to produce, for example, proteins, expression vectors derived from mammals (e.g., pcDNA3 (Invitrogen), pEF-BOS (Nucleic Acids Res. (1990) 18(17), p5322, pEF, pCDM8); insect cells (e.g., "BAC-TO-BAC Baculovirus Expression Systems" (GIBCO-BRL), pBacPAK8); plants (e.g., pMH1, pMH2); animal viruses (e.g., pHSV, pMV, pAdexLew); retroviruses (e.g., pZIPneo); yeasts (e.g., "Pichia Expression Kit" (Invitrogen), pNV11, SP-Q01); and Bacillus subtilis (e.g., pPL608, pKTH50) may be employed besides E.coli.

In order to express in animal cells, such as CHO, COS, and NIH3T3 cells, the vector must have a promoter necessary for expression in such cells, e.g., SV40 promoter (Mulligan R. C. et al. (1979) Nature 277: 108-114), HMLV-LTR promoter, EF1a promoter (Mizushima S. and Nagata S. et al. (1990) Nucleic Acids Res. 18: 5322), CMV promoter, and the like. It is preferable that the vector additionally has a marker gene for selecting transformants (for example, a drug-resistant gene selected by a drug like neomycin, G418, or the like). Examples of vectors with such characteristics include pMAM, pDR2, pBK-RSV, pBK-CMV, pOPRSV, pOP13, and the like.

Furthermore, in order for a host vector system that aims to amplify the copy number in the cells, to obtain a cell strain capable of stable production, a method can be given amplifying the vector with methotrexate (MTX) by incorporating into CHO cells deficient in nucleic acid synthetic pathways, a vector (such as pCHOI) having a DHFR gene that compensates for the deficiency. Alternatively, in order to transiently express a gene, there is a method for transforming COS cells that have the gene for SV40 T antigen on the chromosome with a vector (such as pcD) having the SV40 replication origin. The replication origin may be one derived from a polyomavirus, adenovirus, bovine papilloma virus (BPV), or the like. Also, to amplify the gene copy number in the host cells, selection markers, such as the aminoglycoside transferase (APH) gene, thymidine kinase (TK) gene, E.coli xanthine-guanine phosphoribosyl transferase (Ecogpt) gene, and the dihydrofolate reductase (dhfr) gene may be included in the expression vector.

On the other hand, a gene can be expressed in animals by, for example, inserting the gene into an appropriate vector and introducing this vector into a living cell via the retroviral method, the liposome method, the cationic liposome method, the adenovirus method, or the like. Thus, it is possible to perform gene therapy for phenotypes of diseases caused by mutation or polymorphism of the gene, and such. The vectors used in these methods include, but are not limited to, adenovirus vectors (e.g., pAdexlcw), retrovirus vectors (e.g., pZIPneo), and or the like. General techniques for gene manipulation, such as insertion of a DNA fragment into a vector, can be performed according to conventional methods (Sambrook J. et al. (1989) Molecular Cloning 2nd ed., 5.61-5.63, Cold Spring Harbor Lab. press). Administration to living cells may be performed according to the ex vivo method or the in vivo method.

The host cell into which the vector is introduced is not particularly limited. For example, E.coli, various animal cells and such can be used. The host cell can be used, for example, as a production system to produce and express a protein. Protein production systems include in vitro and in vivo systems. Such production systems using eukaryotic cells or prokaryotic cells can be given as in vitro production systems.

Animal cells, plant cells, and fungi cells can be used as the host cell when using eukaryotic cells. Mammalian cells, for example, CHO, COS, 3T3, myeloma, BHK (baby hamster kidney), HeLa, Vero, amphibian cells e.g., Xenopus oocytes (Valle et al., Nature (1981) 291, 358-340), and insect cells (e.g. Sf9, Sf21, Tn5) are known as animal cells. Among CHO cells, dhfr-CHO (Urlaubb G. and Chasin L.A. (1980) Proc. Natl. Acad. Sci. USA 77: 4216-4220), which are CHO cells deficient in the DHFR gene, and CHO K-1 (Kao F.T. and Puck T.T. (1968) Proc. Natl. Acad. Sci. USA 60: 1275-1281), are particularly preferable. Among animal cells, CHO cells are particularly preferable for large scale expression.

A vector can be introduced into a host cell by, for example, the calcium phosphate method, the DEAE-dextran method, methods using cationic liposome DOTAP (Boehringer-Mannheim), electroporation, lipofection, or the like.

Plant cells originating from Nicotiana tabacum are known as protein producing systems and may be used as callus cultures. As fungal cells, yeast cells such as Saccharomyces, including Saccharomyces cerevisiae, or filamentous fungi such as Aspergillus, including Aspergillus niger, are known.

When utilizing prokaryotic cells, there is a production system using bacterial cells. Bacterial cells, for example, E.coli such as JM109, DH5a , HB101, as well as Bacillus subtilis are known.

A target DNA transforms these cells, and the resulting transformants are cultured in vitro to obtain a protein. Culturing can be performed according to known methods. DMEM, MEM, RPMI1640, or IMDM, or the like, may be used as a culture medium for animal cells with or without serum supplements such as fetal calf serum (FCS). It is preferable that the pH for incubation is approximately 6 to 8. Culturing is typically performed for about 15 to 200 hrs at about 30 to 40 degrees centigrade, and the culture medium may be replaced, aerated, or stirred if necessary.

On the other hand, in vivo protein production system includes, for example, a production system using animal or plant. A target DNA is introduced into an animal or a plant so as to produce proteins in vivo therewithin, and then recovered. With this present invention, these animals and plants are included in the "host".

Animals to be used for the production system described above include mammals and insects. Goats, pigs, sheep, mice, and cattle can be used as mammals (Vicki Glaser, SPECTRUM Biotechnology Applications (1993)). Alternatively, the mammals may be transgenic animals. For instance, a desired DNA may be prepared as a fusion gene with a gene, such as goat β casein gene, that encodes a protein specifically produced into milk. DNA fragments including this fusion gene are injected into goat embryos, which are then introduced back to female goats. Desired proteins are recovered from milk produced by the transgenic goats (those born from the goats that had received the modified embryos) or from their offspring. To increase the amount of milk containing the proteins produced by transgenic goats, appropriate hormones may be administered (Ebert K.M. et al., Bio/Technology (1994) 12: 699-702).

Alternatively, insects, such as silkworm, may be used as the host. When using silkworms, baculoviruses, into which a DNA encoding a desired protein has been inserted, can be used to infect the silkworms, and the desired protein can be recovered from body fluids thereof (Susumu M. et al., Nature (1985) 315, 592-594).

As plant hosts, tobacco can be used, for example. When using tobacco, a DNA encoding a desired protein may be inserted into a plant expression vector, such as pMON 530, which is then introduced into bacteria, such as Agrobacterium tumefaciens. The bacteria are used to infect tobacco, such as Nicotiana tabacum, and the desired polypeptide is recovered from the leaves (Ma J.K. et al. (1994) Eur. J. Immunol. 24, 131-138).

A protein obtained as above can be isolated from the interior or exterior of the host cell (culture medium and such), and purified to a substantially pure homogeneous protein. The method for protein isolation and purification is not limited to any specific method; in fact, any standard method may be used. For instance, column chromatography, filtration, ultrafiltration, salting out, solvent precipitation, solvent extraction, distillation, immunoprecipitation, SDS-polyacrylamide gel electrophoresis, isoelectric point electrophoresis, dialysis, and recrystallization may be appropriately selected and combined to isolate and purify the protein.

Chromatography, such as affinity chromatography, ion-exchange chromatography, hydrophobic chromatography, gel filtration chromatography, reverse phase chromatography, adsorption chromatography and such may be used (Strategies for Protein Purification and Characterization: A Laboratory Course Manual. Ed. Daniel R. Marshak et al., Cold Spring Harbor Laboratory Press (1996)). These chromatographies may be performed using liquid chromatographies, such as HPLC and FPLC. Highly purified proteins can be obtained by the above purification methods.

A protein may be optionally modified or partially deleted by treatment with an appropriate protein-modifying enzyme before or after purification. For example, trypsin, chymotrypsin, lysylendopeptidase, protein kinase, glucosidase and such are used as protein-modifying enzymes.

Antibodies against a protein, which is encoded by a target gene, can be prepared by using proteins obtained as above. The antibodies may take any form, including monoclonal antibodies and polyclonal antibodies. Such form further includes antiserum obtained by immunizing animals such as rabbits with the protein, all classes of polyclonal and monoclonal antibodies, as well as human and humanized antibodies produced by genetic recombination.

A protein used as a sensitizing antigen to obtain antibodies may be derived from any animal species. However, it is preferably from a mammal, such as human, mouse, or rat, more preferably, from a human.

A full-length protein or a partial peptide thereof may be used as a sensitizing antigen with the present invention. A partial peptide may be, for example, an amino (N)-terminus or carboxy (C)-terminus fragment of the protein. Herein, an "antibody" is defined as an antibody that reacts with either the full-length or a fragment of the protein.

For preparing antibodies, a target gene or its fragment may be inserted into a known expression vector used to transform a host cell as described herein. The desired protein or its fragment may be recovered from the exterior or interior of the host cell by any standard method, and may be used as the sensitizing antigen. Alternatively, cells expressing the protein or their lysates, or a chemically synthesized protein may be used as an antigen. Preferably, short peptides are used as antigens by appropriately binding to carrier proteins, such as keyhole limpet hemocyanin, bovine serum albumin, and ovalbumin.

Any mammal may be immunized with the sensitizing antigen. However, preferably, the compatibility with parental cells used for cell fusion is taken into account. In general, animals classified as Rodentia, Lagomorpha, or Primates are used.

Animals classified as Rodentia include, for example, mice, rats, and hamsters. Animals classified as Lagomorpha include, for example, rabbits. Animals classified as Primates include, for example, monkeys of Catarrhini (old world monkeys), such as Macaca fascicularis, rhesus monkeys, sacred baboons, or chimpanzees.

Methods for immunizing animals with sensitizing antigens are well known in the art. Intraperitoneal injection or subcutaneous injection of sensitizing antigens is used for mammals as a standard method. More specifically, the sensitizing antigen may be diluted and suspended with phosphate-buffered saline (PBS), physiological saline, etc. into an appropriate amount. If desired, the antigen suspension may be mixed with an appropriate amount of a standard adjuvant, such as Freund's complete adjuvant, made into an emulsion, and then administered to mammals. Preferably, this is followed by several administrations of the sensitizing antigen mixed with an appropriate amount of Freund's incomplete adjuvant every 4 to 2] days. An appropriate carrier may also be used when immunizing the sensitizing antigen. After the above immunization, the serum is examined for an increase in the amount of desired antibodies by a standard method.

Polyclonal antibodies may be prepared by collecting blood from the immunized mammal after confirming the increase in the levels of desired antibodies in the serum. The serum is separated from the blood by any conventional method. Serum containing a polyclonal antibody may be used as a polyclonal antibody, or if necessary, the fraction containing the polyclonal antibody may be isolated from the serum, and the isolated fraction can then be purified using a protein A or G column, thereby preparing immunoglobulin G or M.

To prepare monoclonal antibodies, immune cells are collected from a mammal immunized with an antigen and checked for an increase in the level of the desired antibodies in the serum as described above, and these cells are subjected to cell fusion. The immune cells used for cell fusion are preferably obtained from the spleen. The other parent cell fused with the above immune cell is preferably a mammalian myeloma cell, and more preferably, a myeloma cell that has acquired a special feature that can be used for selecting fusion cells by a drug.

The above immune cell and myeloma cell may be fused by basically any standard method, such as those described by Milstein et al. (Galfre G. and Milstein C., Methods Enzymol. (1981) 73, 3-46).

Resulting hybridomas obtained by cell fusion may be selected by cultivating in a standard selection medium, such as the HAT medium (medium containing hypoxanthine, aminopterin, and thymidine). The cell culture is typically continued in the HAT medium for a time period that is sufficient to allow all cells, except the desired hybridoma (non-fused cells), to die, usually from several days to several weeks. Then, standard limiting dilution is performed to screen and clone a hybridoma cell producing the desired antibody.

In addition to the above method for immunizing a nonhuman animal with an antigen for preparing a hybridoma, human lymphocytes, such as those infected by the EB virus, may be immunized with a protein, protein-expressing cells, or their lysates in vitro. The immunized lymphocytes are then fused with human-derived myeloma cells having indefinite division ability, such as U266, to yield a hybridoma producing a desired human antibody binding to a protein(Japanese Patent Application Laid-open No. Sho 63-17688).

Subsequently, the hybridomas thus obtained are transplanted into the abdominal cavity of a mouse from which the ascites is collected. The monoclonal antibodies thus obtained can be purified by, for example, ammonium sulfate precipitation or column chromatography using a protein A or protein G column, a DEAE ion exchange column, an affinity column to which a protein encoded by a target gene is coupled, and such. The prepared antibody can be used not only for purifying and detecting the protein encoded by the target gene, but also as a candidate for an agonist or antagonist of the protein. Such an antibody may also be considered to be used for antibody therapy of diseases. To administer the obtained antibody to humans (namely, antibody therapy), human antibodies or humanized antibodies are preferred to reduce immunogenicity.

For example, transgenic animals having a repertory of human antibody genes may be immunized with a protein, protein-expressing cells, or their lysates as antigen. Antibody producing cells are collected from the animals, and fused with myeloma cells to obtain hybridoma, from which human antibodies against the protein can be prepared (see WO92-03918, WO93-2227, WO94-02602, WO94-25585, WO96-33735, and WO96-34096). Alternative to producing antibodies using hybridoma, an immune cell that produces antibodies, such as an immunized lymphocyte, which are immortalized by an oncogene, may be used for preparing antibodies.

The monoclonal antibodies obtained as such can also be recombinantly prepared using genetic engineering techniques (see, for example, Borrebaeck C.A.K. and Larrick J.W., Therapeutic Monoclonal Antibodies, published in the United Kingdom by MacMillan Publishers LTD, (1990)).

A recombinant antibody can be prepared by cloning a DNA encoding the antibody from an immune cell, such as a hybridoma or an immunized lymphocyte producing the antibody; inserting this into an appropriate vector; and introducing the vector into a host cell. The present invention encompasses this recombinant antibody.

According to the present invention, an antibody may be a fragment of an antibody or modified antibody, so long as it binds to a protein encoded by a target gene. For instance, the antibody fragment may be Fab, F(ab)$_2$, Fv, or single chain Fv (scFv) in which Fv fragments from H and L chains are linked by an appropriate linker (Huston J.S., et al.(1988) Proc. Natl. Acad. Sci. U.S.A. 85: 5879-5883). More specifically, treating an antibody with an enzyme, such as papain or pepsin, may generate an antibody fragment. Alternatively, a gene encoding the antibody fragment may be constructed, inserted into an expression vector, and expressed in an appropriate host cell (see, for example, Co M.S. et al., J. Immunol., (1994) 152: 2968-2976; Better M. and Horwitz A.H., Methods Enzymol., (1989) 178: 476-496; Pluckthum A. and Skerra A., Methods Enzymol., (1989) 178,497-515; Lamoyi E., Methods Enzymol., (1986) 121,652-663; Rousseaux J. et al., Methods Enzymol., (1986) 121, 663-669; Bird R.E. and Walker B.W., Trends Biotechnol. (1991) 9, 132-137).

An antibody may be modified by conjugation with a variety of molecules, including polyethylene glycol (PEG). "Antibodies" of the present invention include such modified antibodies. Such modified antibody can be obtained by chemically modifying an antibody. These modification methods arc conventional in the field.

Alternatively, according to the present invention, an antibody may be obtained as a chimeric antibody, comprising a variable region derived from a nonhuman antibody, and the constant region derived from a human antibody, or as a humanized antibody, including the complementarity determining region (CDR) derived from a nonhuman antibody, the framework region (FR) derived from a human antibody, and the constant region, by well-known methods.

Antibodies thus obtained may be purified to homogeneity. Any standard protein separation and purification method may be used for antibody separation and purification according to the present invention. For example, column chromatographies, such as affinity chromatography; filtration; ultrafiltration; salting out; dialysis; SDS polyacrylamide gel electrophoresis; isoelectric point electrophoresis may be appropriately selected and combined to isolate and purify the antibody (Antibodies: A Laboratory Manual. Ed Harlow and David Lane, Cold Spring Harbor Laboratory (1988)). However, the methods are not limited thereto. The concentration of the obtained antibody may be determined by measuring absorbance, by enzyme-linked immunosorbent assay (ELISA), or the like.

Columns used for affinity chromatography include protein A columns and protein G columns. For example, Hyper D, POROS, and Sepharose F.F. (Pharmacia) may be given as columns using protein A columns.

Chromatography other than affinity chromatography includes ion-exchange chromatography, hydrophobic chromatography, gel filtration chromatography, reverse phase chromatography, adsorption chromatography, and such (Strategies for Protein Purification and Characterization: A Laboratory Course Manual. Ed. Daniel R. Marshak et al., Cold Spring Harbor Laboratory Press (1996)). These chromatographies can be conducted using liquid chromatographies, such as HPLC and FPLC.

For example, measurements of absorbance, enzyme-linked immunosorbent assay (ELISA), enzyme immunoassay (EIA), radioimmunoassay (RIA), or immunofluorescence may be used to measure the antigen binding activity of an antibody. In the case of using ELISA, a target protein is applied to a solid state antibody plate, and then a sample containing a desired antibody, such as culture supernatant of antibody producing cells or a purified antibody, is applied. Then, a secondary antibody, which recognizes the primary antibody labeled with an enzyme, such as alkaline phosphatase, is applied. The plate is then incubated. After washing, an enzyme substrate, like p-nitrophenyl phosphate, is added to the plate and absorbance is measured to evaluate the antigen binding activity of the sample. A fragment of a protein, such as a C-terminus fragment or a N-terminus fragment, may be used as the protein. BIAcore (Pharmacia) may be used to evaluate the activity of an antibody.

The above methods allow the detection or measurement of a protein encoded by a target gene, by exposing an antibody to a sample assumed to contain the protein encoded by the target gene in the sample, and detecting or measuring the immune complex formed by the antibody and the protein. Because the measurement method can specifically detect or measure a protein encoded by a gene, the method may be useful in a variety of experiments, tests, diagnoses, and such, in which the protein is used.

The present invention also provides polynucleotides comprising at least 15 nucleotides that are complementary to one strand of a double strand of a target gene or to the complementary strand thereof.

The term "complementary strand" as used herein refers to one strand of a double strand DNA comprising A:T (or A:U for RNA) and G:C base pairs when viewed against the other strand. Furthermore, "complementary" encompasses not only a nucleotide sequence completely complementary to a continuous nucleotide region with at least 15 nucleotides but also a homology of at least 70%, preferably at least 80%, more preferably 90%, and most preferably 95% or more at the nucleotide sequence level. Homology of proteins can be determined using the algorithm described in the literature (Wilbur W.J. and Lipman D.J., Proc. Natl. Acad. Sci. USA, (1983) 80: 726-730).

Such nucleic acids include probes and primers used for the detection and amplification of a target gene; probes and primers for detecting the expression of the gene; and nucleotides and nucleotide derivatives (for example, antisense oligonucleotides, ribozymes, or DNAs encoding them) used for suppressing the expression of the gene. Herein, the detection of a gene also includes the detection of gene mutation. Furthermore, such nucleic acids can be used in the preparation of DNA chips.

If the above polynucleotide is used as a primer, the 3'-region thereof may be the complementary site, and restriction enzyme recognition sites, tag sequences, and such may be attached to the 5'-region. Antisense oligonucleotides include, for example, antisense oligonucleotides that hybridize with any portion of the protein coding region. The antisense oligonucleotide is preferably an antisense of a continuous sequence comprising at least 15 nucleotides or more within the protein coding region. More preferably, the above continuous sequence comprising at least 15 nucleotides or more contains a translation initiation codon.

A derivative or modified form of an antisense oligonucleotide may also be used. The latter form may be prepared by modifying an antisense oligonucleotide with lower alkylphosphonates, such as, methylphosphonate or ethylphosphonate, or with phosphorothioate, or phosphoroamidate.

The antisense oligonucleotide is not restricted to one in which all nucleotides are complementary to the corresponding nucleotides within a given region of a DNA or mRNA. So long as an oligonucleotide can specifically hybridize with a DNA or mRNA encoding a target gene, it may have one or more nucleotide mismatches.

A derivative of an antisense oligonucleotide according to the present invention may act on cells producing a protein encoded by a target gene and bind to a DNA or mRNA encoding the protein. It then may inhibit the expression of the protein by inhibiting its transcription or translation, or by promoting the degradation of mRNA, and thereby inhibiting the function of the protein.

A derivative of an antisense oligonucleotide of the present invention may be mixed with an appropriate base that is inactive against the derivative, and used as a medicine for external application, such as a liniment or poultice. If necessary, it may be mixed with excipients, isotonizing agents, solubilizing agents, stabilizers, preservatives, pain-killers, or such to be prepared as a tablet, powder, granule, capsule, liposome capsule, injectable solution, liquid formulation, nose drops, freeze-dried agent, or such. The above may be prepared according to standard methods.

For treating patients, a derivative of an antisense oligonucleotide according to the present invention may be directly applied to the affected area of a patient, or administered into blood vessels so as to finally reach the affected area. Moreover, the derivative may be encapsulated in antisense-encapsulating materials, such as liposomes, poly-L-lysine, lipid, cholesterol, lipofectin, or their derivatives in order to increase durability and/or membrane permeability.

The dose of a derivative of the antisense oligonucleotides according to the present invention may be appropriately adjusted depending on the patient's conditions, and a preferable amount in the range of, for example, 0.1 to 100 mg/kg, preferably 0.1 to 50 mg/kg, may be administered.

Since the antisense oligonucleotides of the present invention inhibit the expression of a protein encoded by a target gene, they are useful as an inhibitor of the biological activity of the protein. An expression inhibitor containing an antisense oligonucleotide according to the present invention is useful due to its ability to inhibit the biological activity of the protein, and is further useful not only for medicinal application but as a functional analysis tool for the gene.

Test of mutation or expression of a target gene or a protein can be performed using an antibody against the protein encoded by the target gene, or a polynucleotide containing at least 15 nucleotides complementary to one of the chains of the gene or to the complementary strand thereof. When the target gene is related to a disease, testing of the disease can be performed using the antibody or the polynucleotide. The test of a disease according to the present invention includes not only tests of patients expressing symptoms of the disease associated with the mutation of a pathogenic gene, but also tests of the expression level of the pathogenic gene and tests of the mutation of the gene performed to determine whether or not the subject is prone to have the disease due to abnormality in the expression level of the pathogenic gene or due to mutation of the gene. That is, the danger of having the disease is considered to be greatly increased due to abnormalities in the expression of the pathogenic gene and occurrence of mutation in one of the alleles of the pathogenic gene, even when no symptom has risen to the surface. Furthermore, phenotypes other than diseases that have genetic factors can be, for example, tested for the presence or absence of their causative genes, or for the mutation or expression of those genes.

Methods for testing diseases and such using antibodies includes, for example, a method including the step of detecting a protein encoded by a causative gene in a test sample. Specifically, the method for testing with antibodies against proteins encoded by causative genes includes the steps of: (a) making the above-mentioned antibody have contact with a test sample; and (b) detecting binding of the antibody to the test sample. Detection of proteins can be performed by immunoprecipitation using antibodies, Western blotting, immunohistochemistry, ELISA, and such.

For the testing according to the present invention, polynucleotides (probe and primers) complementary to a nucleotide sequence of a transcript or cDNA of a gene and a nucleotide sequence of a genomic DNA sequence (including endogenous transcription regulatory sequence) or to their complementary strands may be used. Incidentally, testing mutation includes a test that specifies "carriers", i.e., those having mutation in one of the alleles.

When used as a primer, polynucleotides are normally 15 to 100 bp, and preferably 17 to 30 bp. There are no limitations on the primer so long as it can amplify at least a portion of a target gene region or a region that regulates its expression. Examples of such regions include, for example, exons, introns, promoters, and enhancer regions of the gene.

On the other hand, as a probe, the polynucleotide normally has a strand length of at least 15 bp or longer if it is a synthetic polynucleotide. Double-stranded DNA obtained from a clone that has been inserted into a vector such as plasmid DNA may also be used as a probe. There are no limitations on the probe so long as it is complementary to the nucleotide sequence of at least a portion of a gene or the region regulating its expression, or to its complementary strand.

The regions to which the probe may hybridize includes, for example, the exon, intron, promoter, and enhancer regions of a gene. When used as probes, the polynucleotide or the double stranded DNA are labeled appropriately, and then used. The labeling methods are, for example, phosphorylating the 5'-terminus of polynucleotide with $^{32}$P using T4 polynucleotide kinase, or incorporating a substrate nucleotide labeled with biotin, fluorescent pigment, isotopes such as $^{32}$P, and such, and using a random hexamer oligonucleotide as a primer and DNA polymerase such as Klenow enzyme (random priming method).

An example of a testing method that utilizes an antibody against a protein encoding a target gene or a polynucleotide containing at least 15 nucleotides complementary to one of the strands of the gene or to its complementary strand, includes a method that includes the step of detecting a transcript of the target gene within a test sample. Such a test method includes methods including the steps of: (a) making the above-mentioned polynucleotide have contact with a test sample, and (b) detecting binding of the polynucleotide to an mRNA in the test sample. Such tests can be performed, for example, by Northern hybridization or RT-PCR.

A test that uses RT-PCR specifically includes (a) synthesizing cDNA from an mRNA in a test sample, (b) performing a polymerase chain reaction using the synthesized cDNA as a template and the polynucleotide of the present invention as a primer, and (c) detecting the DNA amplified by the polymerase chain reaction. Northern hybridization and RT-PCR can be performed by well-known genetic engineering techniques. Also, detection using a DNA chip or a DNA micro-array is possible.

In addition, test of diseases and such may be performed by detecting mutations or polymorphisms in target genes. Specifically, such tests can be performed by detecting mutations or polymorphisms in the transcription regulatory region or encoding region of a target gene.

According to an embodiment of such testing methods, the nucleotide sequence of a target gene from a subject is directly determined. For example, a portion or all of a target gene from a subject (for example, regions including exons, introns, promoter, and enhancer) may be amplified by PCR (Polymerase Chain Reaction) and such, using the above-mentioned nucleotide as a primer, DNA isolated from a subject as a template, and the nucleotide sequence thereof may be determined. Then, the determined sequence may be compared to the sequence of a gene derived from a control subject (e.g., healthy person, etc.) to accomplish the test.

As a method for testing according to the present invention, various methods are used in addition to the method for directly determining the nucleotide sequence of DNA derived from the subject. An embodiment of the test methods includes the steps of: (a) preparing a DNA sample from a subject, (b) amplifying the subject-derived DNA using the polynucleotide of this invention as a primer, (c) dissociating the amplified DNA into single-stranded DNA, (d) separating the dissociated single stranded DNA on a non-denaturing gel, and (e) comparing the mobility of the separated single-stranded DNA on the gel with that of a control subject.

An example of such methods is a single-strand conformation polymorphism (PCR SSCP) method (Cloning and polymerase chain reaction-single-strand conformation polymorphism analysis of anonymous Alu repeats on chromosome 11. (1992 Jan 1) Genomics 12(1): 139-146; Detection of p53 gene mutations in human brain tumors by single-strand conformation polymorphism analysis of polymerase chain reaction products. (1991 Aug 1) Oncogene 6(8): 1313-1318; Multiple fluorescence-based PCR-SSCP analysis with postlabeling. (1995 April 1) PCR Methods Appl. 4(5): 275-282).

This method is relatively simple and has the advantage of requiring the smallest amount of sample. Therefore, it is especially preferable when screening many DNA samples. Its principles are as follows. When a double-stranded DNA fragment is dissociated into single strands, each strand forms an independent higher-order structure according to its nucleotide sequence. When this dissociated DNA strand is electrophoresed on a polyacrylamide gel that does not contain a denaturant, depending on the difference in each higher-order structure, complementary single-stranded DNA having the same length of nucleotides move to different positions. The higher-order structure of such single-stranded DNA changes even with replacement of a single nucleotide, and indicates different mobility in the polyacrylamide gel electrophoresis. Therefore, by detecting this change in mobility, existence of mutation in the DNA fragment, such as point mutation, deletion, or insertion, can be detected.

Specifically, to begin with, a whole target gene, or a portion of it, is amplified by PCR and such. Normally, the amplified range preferably has a length of approximately 200 to 400 bp. The amplified region includes all exons and all introns of the gene and also, promoter and enhancers of the gene. During gene fragment amplification by PCR, synthesized DNA fragments are labeled by performing PCR using a primer labeled with isotopes, such as $^{32}P$ or with fluorescent pigments, biotin, and such, or by adding a substrate nucleotide labeled with isotopes such as $^{32}P$, or with fluorescent pigments, biotin, and such into the PCR reactive solution. Otherwise, labeling can be performed by adding a substrate nucleotide labeled with isotopes, such as $^{32}P$, or with fluorescent pigments, biotin, and such to a synthesized DNA fragment using Klenow enzyme and such, after PCR.

The labeled DNA fragment obtained as such is denatured by heating and such, and electrophoresis is performed using a polyacrylamide gel that does not contain denaturants such as urea. Here the conditions for DNA fragment separation can be improved by adding an appropriate amount (approximately 5% to 10%) of glycerol to the polyacrylamide gel. Electrophoretic conditions change with properties of each DNA fragment, but normally, it is performed at room temperature (20°C to 25°C). When a favorable separation cannot be achieved, the temperature that gives the most appropriate mobility is tested to be between 4°C to 30°C.

After electrophoresis, mobility of the DNA fragment is detected by autoradiography using an X-ray film, a scanner detecting fluorescence and such, and then analyzed. When a band having a difference in mobility is detected, this band is directly cut out from the gel and then re-amplified by PCR, and by direct sequencing, the existence of mutation can be confirmed. Also, even when labeled DNA is not used, by staining the gel after electrophoresis with ethidium bromide or by silver staining and such, bands can be detected.

Another embodiment of the testing method of this invention includes the steps of: (a) preparing a DNA sample from a subject, (b) amplifying the subject-derived DNA using a polynucleotide of this invention as a primer, (c) cleaving the amplified DNA, (d) separating the DNA fragments according to their size, (e) hybridizing the detectably labeled polynucleotide of this invention as a probe to the separated DNA fragment, and (f) comparing the size of the detected DNA fragment with that of a control subject.

Examples of such methods include methods using restriction fragment length polymorphism (RFLP), PCR-RFLP, and such. Normally, restriction enzymes are used as enzymes to cleave DNA. Specifically, when mutation or polymorphism exists at the restriction enzyme recognition site, or when there is a nucleotide insertion or deletion in the DNA fragment formed by restriction enzyme treatment, the size of the fragment formed after restriction enzyme treatment changes compared to that of a control (healthy subject). Amplifying this portion containing the mutation or polymorphism by PCR, and then treating with each restriction enzyme enables the detection of these mutations or polymorphisms as differences in band mobility after electrophoresis. Otherwise, chromosomal DNA is treated with these restriction enzymes, and after electrophoresis, by performing Southern blotting using the polynucleotide described above as a probe, the presence or absence of mutation or polymorphism can be detected.

The restriction enzyme to be used can be appropriately selected depending on each region to be tested. In this method, in addition to genomic DNA, RNA prepared from a subject may be made into cDNA by reverse transcriptase, and after cleaving it in its original form by a restriction enzyme, Southern blotting can be performed. Also, using this cDNA as a template, a portion of or a whole target gene can be amplified by PCR, and after cleaving it with restriction enzyme, their difference in mobility can be investigated.

Furthermore, instead of using DNA prepared from a subject, a similar detection is possible using RNA as well. Such a method includes the steps of: (a) preparing an RNA sample from a subject, (b) separating the prepared RNA according to size, (c) hybridizing the polynucleotide of this invention, that has a detectable label as a probe, to the separated RNA, and (d) comparing the size of the detected RNA with that of a control. An example of a specific method includes electrophoresing RNA prepared from a subject, performing Northern blotting using the polynucleotide described above as a probe, and detecting differences of mobility.

An embodiment of the test methods includes the steps of: (a) preparing a DNA sample from a subject, (b) amplifying the subject-derived DNA using the polynucleotide of this invention as a primer, (c) dissociating the amplified DNA on a gel having a gradually-increased concentration of a DNA denaturant, and (d) comparing the mobility of the dissociated DNA on the gel with that of a control subject.

An example of such a method is denaturant gradient gel electrophoresis (DGGE). The whole target gene, or a portion thereof, is amplified by PCR using the primer as described above, and such. This is then electrophoresed on a polyacrylamide gel in which the concentration of a denaturant such as urea gradually becomes higher in the gel as the material moves, and this is compared to that of a control subject (such as a healthy subject). For a DNA fragment in which mutation exists, the DNA fragment becomes single stranded at a position of lower denaturant concentration, and the rate of movement becomes extremely slow. Therefore, by detecting this difference in mobility, the presence or absence of mutation of polymorphism can be detected.

Apart from these methods, for the purpose of detecting mutations only at a particular position, allele specific oligonucleotide (ASO) hybridization can be used. An oligonucleotide containing a sequence in which a mutation is thought to exist is prepared, and when this is hybridized with sample DNA, if mutation exists, the efficiency of hybrid formation decreases. This can be detected by Southern blotting or by a method that utilizes the property of quenching by intercalation of a specialized fluorescent reagent into a gap in the hybrid, or such a method.

Also, detection by a ribonuclease A mismatch cleavage method is possible. Specifically, a whole target gene, or a portion thereof, is amplified by PCR and such, and the product is hybridized with labeled RNA prepared from a target gene fragment and such inserted in a plasmid vector, and such. In the portion in which mutation exists, the hybrid takes a single-stranded structure, therefore, this portion is cleaved by ribonuclease A, and by detecting this with autoradiography, and such, existence of mutation can be detected.

(Examples)

(Example 1)

(1) Sampling of DNA samples

To make a genetic analysis using blood samples of patients suffering from each disease and healthy subjects, which are controls, collecting blood of 10 to 20 ml from 464 Japanese healthy subjects who have agreed to participate in the study, 306 patients with rheumatoid arthritis, and 118 patients with psoriasis vulgaris is carried out, and DNA samples are then collected.

It is noted that this study was made upon examination and approval by the Japan Biological Information Consortium Committee for Ethical Examination, Tokai University Research of Human Genome and Genetic Analysis Committee for Ethical Examination, University of Tokyo Graduate School of Medicine and Faculty of Medicine, Research Committee for Ethical Examination, Juntendo University Faculty of Medicine Research Ethical Committee.

(2) Preparation of DNA samples

Preparation of DNA samples is carried out in conformity with the following procedure. Extraction and purification of the genome DNA from the collected blood is carried out using QIAamp DNA Blood Maxi Kit (QIAGEN). Considering a possible influence to amplification efficiency for PCR, T.E. (10 mM Tris-HCl, 0.1 mM EDTA), which is one tenth the normal EDTA concentration, is used to extract the genome DNA from columns. Once extraction is accomplished, agarose gel electrophorsis is carried out so as to determine that decomposition of DNA does not occur, and also to find the purity based on the measured absorbance or 260/ 280 ratio.

Measurement of DNA and preparation of mixed DNA (pooled DNA) solution are carried out in conformity with the following procedure. A fluorescence plate reader and the fluorescent pigment PicoGreen reagent (Molecular Probes), which stains a double strand DNA specifically, are used to measure DNA. ? phage DNA attached to the PicoGreen reagent is used as standard samples to be used for concentration measurement, which include five-point dilution sequences of 10, 30, 100, 300, and 1000 pg/mL. The genome DNA of each individual is diluted into 1/ 400, and measurements are then carried out three times for the respective sequences. From the results of the three-time measurements, a total of three combinations of two are made, and average values, S.Ds. and C.Vs. are calculated for the respective combinations. The combination with a C.V. of 5% or less and being the lowest value is selected, and the corresponding average is then used as the final DNA concentration. If the respective C.Vs. for all of the three combinations exceeds 5%, measurement of the DNA is carried out repeatedly until a combination with 5% or less is found.

Based on the DNA concentration determined by such operation and measurement, fixed DNA amounts from DNA samples of 125 subjects are mixed together, and T.E.(10 mM Tris-HCl, 0.1 mM EDTA) is added thereto to obtain an 8 ng/$\mu$L pooled sample. To perform the first and the second screening, two sets of such pooled DNA sample for the 125 subjects are prepared for each patient group and healthy subject group. It is noted that the sex ratio for the 125 subjects in each set is selected so that it can be equal between the pooled DNA samples for each patient group and those for the healthy subject group, and the age distribution is selected so that it can also be almost equal between them in the 10-year-old class.

(3) Preparation of DNA sequence fragment group including microsatellite genetic polymorphism markers (Pooled DNA genotyping)

A set of a forward primer and reverse primer including 15 to 100 nucleotides, preferably 15 to 25 nucleotides, more preferably 18 to 22 nucleotides, which are obtained from the DNA sequence made up of the nucleotide sequences of sequence numbers 1 to 27088, is used to amplify a DNA sequence fragment group including microsatellite genetic polymorphism markers. The forward primer with its 5'-terminus fluorescently labeled with 6-FAM or HEX(PE Biosystems, Foster City, CA) is used whereas the reverse primer is not labeled.

Preparation of a reactive solution for PCR is performed in conformity with the following procedure. Preparation is made such that a 24 ng pooled DNA sample (i.e., 3 $\mu$L x 8 ng/ $\mu$L), 2 $\mu$L/ 10x buffer (100 mM Tris-HCl, pH 8.3, 500 mM KCl, and 15 mM MgCl2), 2 pmol forward primer and reverse primer, and 0.5 U AmpliTaq DNA polymerase (Applied Biosystems) are included in a 20 $\mu$L reactive solution. The PCR cycle includes: one cycle consisting of processing carried out for 5 minutes at 96 °C, 1 minute at 56 °C, and 1 minute at 72 °C, and 40 cycles consisting of processing carried out for 45 minutes at 96 °C, 45 seconds at 57 °C and 45 seconds at 72 °C.

The obtained PCR products are diluted with ultra pure water into 1/ 20 or 1/ 40, dried by a vacuum pump or an evaporator, and then dissolved in a buffer containing formamide (Applied Biosystems) and DNA size marker GS500 ROX (Applied Biosystems). Afterwards, they are heat-treated for 5 minutes, and then electrophoresed by DNA analyzer ABI 3700 followed by being subjected to determining, using the GeneScan analysis software (Applied Biosystems), the size of each fluorescence signal peak derived from the PCR products. After this operation, each peak size and height are measured using the PickPeak software so as to make data for analysis.

(4) Statistical calculation

Regarding each MS genetic polymorphism marker included DNA sequence, in order to compare the estimated allele frequencies obtained from the pooled DNA samples for a patient group and pooled DNA samples for a healthy subject group so as to make a genetic correlation analysis, statistical processing based on the chi-square test and the Fisher test is performed in conformity with the following procedure.

The total of the heights of fluorescence signal peaks derived from the detected PCR products is calculated for each gene locus (MS genetic polymorphism marker). With this as the denominator, the ratio of each allele peak height is calculated to be the gene frequency for each allele within a group. In the statistical processing, an allele number for each allele within a group is found by multiplying the total allele number for the sample included in the pooled DNA sample, and using this allele number, a test regarding the difference among the allele frequencies of the healthy subject group and the patient group is carried out.

Moreover, for genetic correlation analysis using pooled DNA samples based on the above-described method, 2 x 2 and 2 x m divided tables are made to calculate statistic values through the chi-square test and the Fisher test. These results allow optimization of the genetic correlation analysis using pooled DNA samples. Methods for performing a quick analysis are thus studied and corresponding software is developed.

(Example 2) Specification of susceptibility gene for psoriasis vulgaris (1)

Using the method described in Embodiment 1, genome correlation analysis of the susceptibility gene for psoriasis vulgaris is carried out. This screening allows identification of the gene locus extending from the centromeric MICB gene of the sixth chromosome to the telomeric HLA-F gene, and identification of 758 microsatellite loci existing from 2'- to 5'-nucleotide within the 1.8 Mb HLA class I region including the HLA-B and the HLA-C gene. This fact corresponds to conventional reports (Mizuki N. et al. (1997) Genomics, 42, 55-66; Shiina T. et al. (1998) Genomics, 47, 372-382; Shiina T. et al. (1999) Immunol Rev., 167, 193-199; Tamiya G. et al. (1998) Tissue Antigens, 51, 337-346).

Among these microsatellites, 70 are analyzed for their polymorphism content in the Japanese population. Of these, 38 are found to be highly informative with an average of 66% heterozygosity and an average of 8.9 alleles (Tamiya G. et al. (1998) Tissue Antigens, 51, 337-346; Tamiya G. et al. (1999) Tissue Antigens, 54: 221-228). These 38 microsatellite repeats are selected for high resolution mapping.

Combined with the seven previously known polymorphic genes and microsatellites [MICB, MICA, HLA-B, HLA-C, HLA-A, MIB (Grimaldi M. C. et al. (1996) Hum. Immunol., 51, 89-94), and D6S265 (Weissenbach J. et al. (1992) Nature, 29, 794-801)], a total of 45 informative genetic markers, i.e. one every 41.1 kb, are defined within the HLA class I region. It is considered that this high density polymorphism marker allows acquisition of exact information regarding the haplotype analysis and mapping analysis of psoriasis vulgaris HLA class I related diseases.

(Example 3) Specification of susceptibility gene for psoriasis vulgaris (2)

Next, to determine the definite position of the causative gene for psoriasis vulgaris within the HLA class I region, association analyses are conducted using 11 of these 38 repeats.

These eleven microsatellites are selected so that they can be distributed around the HLA-C gene locus of the sixth chromosome at the rate of one in 100 kb.

Correlation analysis targets the psoriasis vulgaris patient group and the healthy subject group described in Embodiment 1. The patient group consists of 51 males and 25 females with an average onset age of 33 years (SD= 15.3).

To determine the number of repeat units of the eleven genetic polymorphism microsatellite loci (genotype of microsatellite allele), the reverse primer and forward primer with 20 to 28 nucleotides are synthesized from the MS genetic polymorphism marker included DNA sequence. It is noted that this synthesizing uses the fluorescent reagent 6-FAM, HEX, or TET (PE Biosystems (presently Applied Biosystems), Foster City, CA) so as to label the 5'-terminus of the forward primer. Table 1 shows eleven sets of the primers, i.e., twenty-two in all.

Table 1

Microsatellite markers used for correlation analysis

| Micro-Satellite | Localization | Repeat unit | PCR primer |
|---|---|---|---|
| C1_2_A | Tel. ( 0 kb)/MICB<br>Cen.(89 kb)/MICA | (CA)₂₀ | CA: AATAGCCATGAGAAGCTATGTGGGGGAG<br>TG: CTACCTCCTTGCCAAACTTGCTGTTTGTG |
| C1_4_1 | Tel. (40 kb)/MICA<br>Cen.( 6 kb)/HLA-B | (CAAA)₈ | CAAA: CGAGAACAACTGGGAGGACTG<br>TTTG: GACAGTCCTCATTAGCGCTGAGG |
| C1_2_5 | Tel. (62 kb)/HLA-B<br>Cen.(19 kb)/HLA-C | (CA)₄AA(CA)₂₀ | CA: CAGTAGTAAGCCAGAAGCTATTAC<br>TG: AAGTCAAGCATATCTGCCATTTGG |
| C1_4_3 | Tel. (26 kb)/HLA-C<br>Cen.(71 kb)/OTF3 | (GGAA)₁₃ | GGAA: TAGAAAACGCAATCTCGGCC<br>TTCC: CTGGATTAACCTGGAGACTC |
| C1_3_1 | Tel. (27 kb)/HLA-C<br>Cen.(69 kb)/OTF3 | (TTG)₈ | TTG: CAGTGACAAGCACCTGGCAC<br>CAA: GCCAGATGTGGTGGCATGC |
| C1_2_6 | Tel. (85 kb)/HLA-C<br>Cen.(11 kb)/OTF3 | (TA)₁₇ | TA: TGTTCAGACCTCTTCCTGCC<br>AT: GACTAGCTCTTGACTACTTG |
| C1_3_2 | Tel. (37 kb)/OTF3<br>Cen.( 7 kb)/S | (TAA)₁₆ | TAA: TAGGGATGGTCCCAAACGTG<br>TTA: CCCGTGCAGGACTGATCTCC |
| C2_4_4 | Tel. (80 kb)/S | (GAAA)₆AAAAA(GAAA)₃ | GAAA: GGCTTGACTTGAAAGTCAGAGACC<br>TTTG: TTATCTACTTATAGTCTATCACGG |
| C4_2_12 | Tel. (75 kb)/DDR<br>Cen.(89 kb)/TUBB | (CA)₁₃ | CA: GAGCCACGGAGAGTCTCCCTTTATC<br>TG: TCCAGGAACTGTGACTAGTAAGAAC |
| C4_2_25 | Tel. (69 kb)/TUBB<br>Cen.(47 kb)/HSGT260 | (TG)₁₄ | TG: TCTTCTGTGCAAGCAATGCACTGTAC<br>CA: ATGTTACTTTTAGAAGATAACACTC |
| C3_2_11 | Tel. (50 kb)/HLA-E<br>Cen.(21 kb)/MICC | (GA)₂₂TA(GA)₈ | GA: AGATGGCATTTGGAGAGTGCAG<br>TG: TGCTTACAGCAGAGATATGTGG |

Eleven selected microsatellite genetic polymorphism markers and PCR primer sequence numbers are listed here. The numbers on the left denote the sequence numbers for the forward primer, while the numbers on the right denote the sequence numbers for the reverse primer.

| C1-2-A; | Sequence number 27089: | Sequence number 27090 |
|---|---|---|
| G1-4-1; | Sequence number 27091: | Sequence number 27092 |
| C1-2-5; | Sequence number 27093: | Sequence number 27094 |
| C1-4-3; | Sequence number 27095: | Sequence number 27096 |
| C1-3-1; | Sequence number 27097: | Sequence number 27098 |
| C1-2-6; | Sequence number 27099: | Sequence number 27100 |
| C1-3-2; | Sequence number 27101: | Sequence number 27102 |
| C2-4-4; | Sequence number 27103: | Sequence number 27104 |
| C4-2-12; | Sequence number 27105: | Sequence number 27106 |
| C4-2-25; | Sequence number 27107: | Sequence number 27108 |
| C3-2-11; | Sequence number 27109: | Sequence number 27110 |

The PCR reactive mixture is prepared to contain 50 ng genomic DNA, 2 μl dNTP (2.5 mM each), 2 μl/ 10x buffer (100 mM Tris-HCl, pH 8.3, 500 mM KCl, 15 mM MgCl₂), and 20 pmol forward and reverse primers as well as 0.5 U Takara recombinant Taq polymerase (Takara Shuzo, Kyoto, Japan) in a total volume of 20 μl. After initial denaturation for 5 minutes at 96 °C, processing is carried out using an automated thermal cycler (Takara Shuzo) for 30 cycles consisting of 1 minute at 96 °C, 30 seconds at 55 °C and 45 seconds at 72 °C, with a final extension of 4 minutes at 72 °C. The amplified products are denatured for 5 minutes at 100° C, mixed with formamide-containing stop buffer, applied with a size standard marker of GS500 Tamra (PE Biosystems) to the starting position of each lane, and run on a 4% polyacrylamide denaturing sequencing gel containing urea in an automated DNA sequencer. Fragment sizes are determined automatically using the GeneScan software (PE Biosystems).

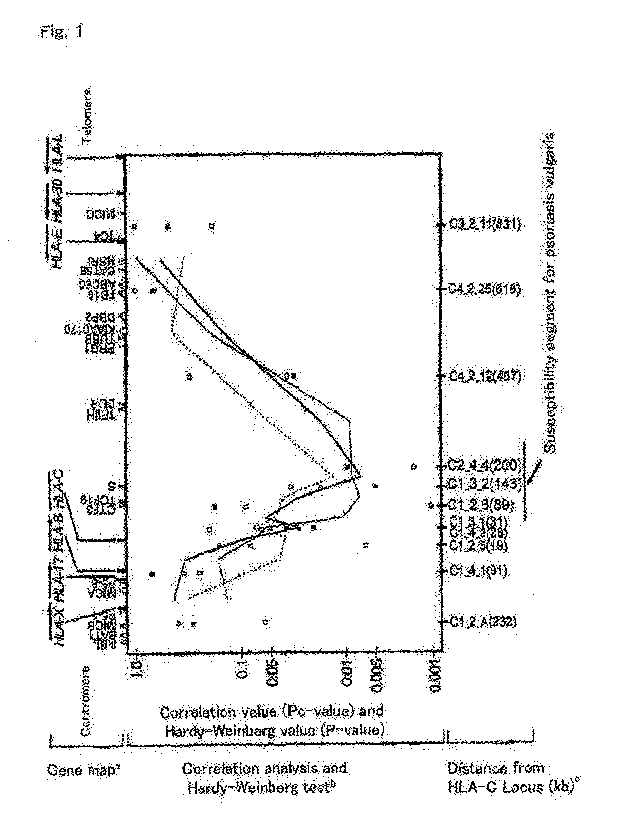

The order of the MS genetic polymorphism markers from the centromeric towards the telomeric is C1_2_A, C1_4_1, C1_2_5, C1_4_3, CC3_1, C1_2_6, C1_3_2, C2_4_4, C4_2_12, C4_2_25, and C3_2_11 (FIG. 1). Repeat units are determined from sequencing data previously obtained by the present inventors (Shiina T. et al. (1999) Immunol. Rev., 167: 193-199). All MS genetic polymorphism markers are established by Tamiya et al. (Tamiya G. et al. (1998) Tissue Antigens, 51, 337-346; Tamiya G. et al. (1999) Tissue Antigens, 54: 221-228).

"Tel" indicates the telomeric, and "Cen.", the centromeric. The PCR primer sequences in order from top of the list are numbered sequence number 27089 to sequence number 27110.

Allele frequencies are estimated by direct counting. The test of significance for the distribution of alleles between the patients and the controls is conducted through the chi-square test, which performs correction for continuity, and Fisher's exact test (P-value test). The P-value is corrected by multiplying by the number of microsatellite alleles observed in each locus (Pc). A level of Pc <0.05 is accepted as statistically significant. The odds ratio for the risk of psoriasis vulgaris is calculated from the 2x 2 contingency table. The exact P-value test of the Hardy- Weinberg proportion for multiple alleles is simulated based on the Markov chain method within the Genepop software package (Tamiya G. et al. (1998) Tissue Antigens, 51, 337-346; Tamiya G. et al. (1999) Tissue Antigens, 54: 221-228; Guo S. W. and Thompson E. A. (1992) Biometries, 48: 361-372). The Markov chain method has the advantage of giving a complete enumeration for testing the Hardy- Weinberg proportion in cases where the number of alleles as well as sample size are small. When the number of alleles is below 5, the exact P-value is calculated by the complete enumeration method. A level of P <0.1 is accepted as statistically significant for the Hardy-Weinberg equilibrium test.

In the patients, the phenotype frequency of HLA-Cw6 (8/76 patients, 10.5%) is significantly increased, with a Pe-value of 0.02 (odds ratio= 15.88). As shown in Table 2, alleles showing statistically significant differences in Pc-values below 0.05 in the patient group are found at the four microsatellite loci: allele 303 ($X^2$= 12.62, Pc= 0.0015) in C1_2_6; 357 ($X^2$= 7.91, Pc= 0.0034) in C1_8_2; 255 and 259 ($X^2$= 9.53, Pc= 0.0012 and $X^2$= 11.58, Pc= 0.0022, respectively) in C2_4 4; and 223 ($X^2$= 7.59, Pc= 0.036) in C4_2_12. X means Chi in Greek-letters. Alleles in each microsatellite genetic polymorphism marker are named on the basis of the length of the amplified fragment. The most significant correlation is obtained for the allele 303 in the C1_2_6 locus. All four microsatellites in the segment from the loci C1_2_6 to C4_2_12 exhibits statistically significant differences in their allele frequencies between the patients and controls (Table 2 and FIG. 1).

Table 2

| | | | | | | | | |
|---|---|---|---|---|---|---|---|---|
| Statistically significant alleles that correlate with psoriasis vulgaris | | | | | | | | |
| Loci | Distance from HLA-C a) | No. of alleles | Allele | Odds ratio | CI of odds ratio | $X^3$ | P-value b) | Pe-value e) |
| C1_2_A | 232kb(c) | 10 | 242 | 0.44 | 0.24-0.81 | 6.97 | 0.00059 | 0.059 |
| C1_4_1 | 91kb(c) | 5 | 221 | 0.59 | 0.31-1.11 | 2.66 | 0.069 | 0.345 |
| C1_2_5 | 19kb(c) | 14 | 216 | 2.58 | 1.59-5.08 | 7.54 | 0.0057 | 0.0798 |
| C1_4_3 | 29kb(t) | 17 | 467 | 0.16 | 0.04-0.59 | 7.52 | 0.0037 | 0.0629 |
| G1_3_1 | 31kb(t) | 4 | 291 | 0.49 | 0.27-0.89 | 5.57 | 0.013 | 0.052 |
| C1_2_6 | 89kb(t) | 8 | 303 | 0.27 | 0.09-0.50 | 12.62 | 0.00019 | <u>0.00152</u> |
| C1_3_2 | 143kb(t) | 8 | 357 | 2.37 | 1.30-4.32 | 7.91 | 0.0042 | <u>0.0336</u> |
| C2_4_4 | 200kb(t) | 6 | 255 | 2.76 | 1.45-5.25 | 9.53 | 0.002 | <u>0.012</u> |
| C2_4_4 | | | 259 | 0.24 | 0.11-0.55 | 11.58 | 0.00037 | <u>0.00222</u> |
| C4_2_12 | 457kb(t) | 9 | 223 | 0.33 | 0.73-0.15 | 7.59 | 0.004 | <u>0.0036</u> |
| C4_2_25 | 618kb(t) | 7 | 271 | 5.06 | 0.56-45.1 | 2.11 | 0.2 | 1 |
| C3_2_11 | 831kb(t) | 17 | 209 | 0.55 | 0.26-1.17 | 2.43 | 0.082 | 1 |

a) (c), centromeric HLA-C gene; (t), telomeric HLA-C gene.

b) Determined by Fisher's exact test.

c) Corrected by multiplying by the number of microsatellite alleles observed in each locus. Pe-values below 0.05, accepted as statistically significant, are underlined.

The exact test of Hardy-Weinberg proportion is also carried out for the above 11 microsatellite genetic polymorphism markers by the Markov chain method (Guo S. W. and Thompson E. A. (1992) Biometrics, 48: 361-372) in terms of deviation from Hardy-Weinberg proportions (the probability test) and heterozygote deficiency against the null hypothesis of Hardy-Weinberg equilibrium (Raymond M. and Rousset F. (1995) J. Hered., 86: 248-249; Rousset F. and Raymond M. (1995) Genetics, 140: 1413-1419). All of the tested 11 MS genetic polymorphism markers follow the Hardy-Weinberg equilibrium in the healthy controls (P >0.25), as expected. In contrast, five loci deviate significantly from the Hardy-Weinberg equilibrium in the patient group (P <0.1: C1_2_5, C1_3_1, C1_2_6, C1_3_2, and C2_4_4), as listed in Table 3. Furthermore, five loci show a significant decrease in heterozygotes (P <0.1: C1_4_3, C1_3_1, C1_3_2, C2_4_4, and C4_2_12). On the other hand, no increase in heterozygotes is observed for any MS genetic polymorphism markers. It must be noted that the three microsatellite loci (C1_3_1, C1_3_2, and C2_4_4) in the segment from C1_3_1 to C2_4_4 displays significant P-values in both probability and heterozygote deficiency tests (Table 3 and FIG. 1). In particular, in C1_3_2 and C2_4_4, highly significant P-values are obtained in both of these tests.

Table 3

Exact test of the Hardy-Weinberg proportion for microsatellites

| Loci | HW[a] | SE | Hetero.[b] | SE | Ex. hetero.[c] | Ob. hetero.[d] |
|------|------|------|----------|------|-------------|-------------|
| C1_2_A | 0.3939 | 0.0043 | 0.2881 | 0.0042 | 0.801 | 0.763 |
| C1_4_1 | 0.2443 | 0.0016 | 0.7024 | 0.0019 | 0.633 | 0.613 |
| C1_2_5. | 0.0063 | 0.0006 | 0.1599 | 0.0042 | 0.883 | 0.842 |
| C1_4_3 | 0.1968 | 0.0065 | 0.0362 | 0.0028 | 0.89 | 0.829 |
| C1_3_1 | 0.0286 | - | 0.0203 | - | 0.561 | 0.461 |
| C1_2_6 | 0.0889 | 0.0023 | 0.1776 | 0.0027 | 0.676 | 0.579 |
| C1_3_2 | 0.0172 | 0.0005 | 0.0051 | 0.0003 | 0.848 | 0.75 |
| C2_4_4 | 0.0007 | 0.0004 | 0.0093 | 0.0003 | 0.655 | 0.553 |
| C4_2_12 | 0.3006 | 0.0052 | 0.0303 | 0.0013 | 0.679 | 0.635 |
| C4_2_25 | 0.666 | 0.0041 | 0.6684 | 0.007 | 0.466 | 0.481 |
| C3_2_11 | 0.1837 | 0.0057 | 0.4787 | 0.0078 | 0.9 | 0.895 |

In the above table, the exact P-value is estimated by the simulations based on the Markov chain method with the following parameters: dememorization number = 1000; number of batches = 400; number of iteration per batch = 8000. When the number of alleles is below 5, the exact P-value is calculated by the complete enumeration method. Pc-values below 0.1, accepted as statistically significant, are underlined. SE, standard error.

    a) Deviation from Hardy-Weinberg proportions (probability test).
    b) Heterozygote deficiency against the null hypothesis of the Hardy-Weinberg equilibrium.
    c) Expected helerozygote frequency in the patient population.
    d) Observed heterozygote frequency in the patient population.

As indicated above, four of the analyzed microsatellites (C1_2_6, C1_3_2, C2_4_4, and C4_2_12) in the segment from C1_2_6 to C4_2_12 display statistically significant differentiation between the patients and controls (Table 2 and FIG. 1). Moreover, the three microsatellite loci (C1_3_1, C1_3_2, and C2_4_4) in the segmentfrom C1_3_1 to C2_4_4 display significant deviation in both probability and heterozygote deficiency tests (Table 3 and FIG. 1). In particular, in C1_3_2 and C2_4_4, highly significant P-values are obtained in both of these tests.

Although the mode of inheritance of psoriasis is unclear, Table 2 makes it clear that the frequency of heterozygotes at the five microsatellite loci in the patients is lower than the expected suggests a recessive HLA trait for this disease, although the genetic penetrance is not high. Collectively, it can be concluded that the 111 kb segment from C1_2_6 (89 kb telomeric HLA-C) to C2_4_4 (200 kb telomeric HLA-C) is the common area critical for psoriasis vulgaris at a confidence level of more than 95%, as assessed by both statistical methods for allelic distribution and deviation on the Hardy-Weinberg equilibrium (FIG. 1).

It must be emphasized that these two independent statistical methods, of which the former deals with the data of patients and controls and the latter only with the data of patients, reveals an almost identical critical segment for psoriasis vulgaris. This result is consistent with previous mapping data which showed the susceptibility gene for psoriasis vulgaris to be residing on the telomeric HLA-C gene, based on a transmission/disequilibrium test (TDT) and parametric linkage analysis using the HLA class I (HLA-A, -B, and -C) and class II (HLA-DRB1 and -DQB1) alleles in the patients (Jenisch S. et al. (1998) Am. J. Hum. Genet., 63, 191-199).

(Example 4) Analysis of susceptibility gene for rheumatoid arthritis.

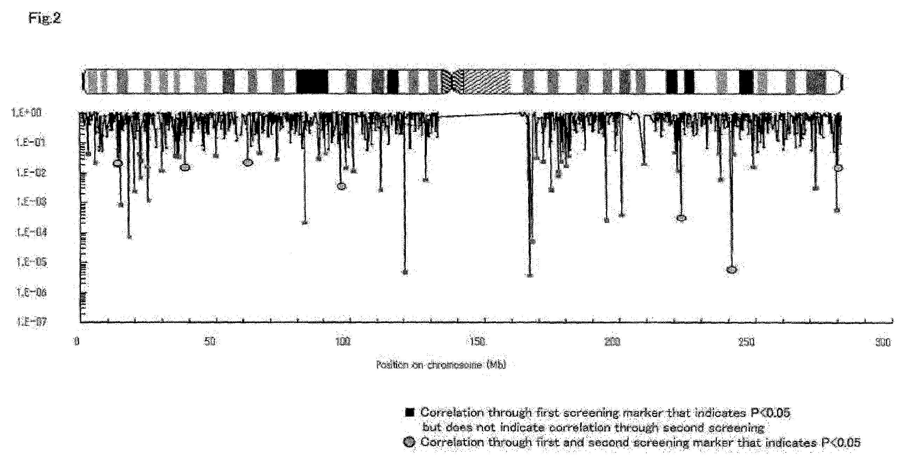

In the first step of identifying the susceptibility gene for rheumatoid arthritis in which genetic factors are suggested to be involved, a genetic correlation analysis is carried out throughout the genome. Considering the sampling structure for age and sex of patients and healthy subjects, the pooled DNA reagents for analysis of the susceptibility gene for rheumatoid arthritis are adjusted. The sex ratio for the reagents collected from the rheumatoid arthritis patients before the test has begun is 0.212 for men to 0.788 for women. In order to perform the second screening, two pooled DNA reagents (i.e., 125 x2) are prepared in accordance with the above sex ratio. The age distribution for this case is illustrated in the ten-year-old class(FIG. 2). The average ages for the reagent pool of to-be-compared healthy subjects is shown below:

| | | | | | | | | |
|---|---|---|---|---|---|---|---|---|
| <Men> | Average age: | 44.3 | SD: | 11.8 | Sample number: | 52 |
| <Women> | Average age: | 39.1 | SD: | 12.9 | Sample number: | 198 |

Since there is no information regarding onset ages for patients, certain references are referred. The age distribution is surveyed (since the average age is unknown, the age of 45 is given), and in conformity therewith, the pooled DNA reagents for the healthy subjects are prepared.

To begin with, screening the pooled DNA samples for the patients and healthy subjects is carried out, as described in Embodiment 1, using primers corresponding to MA genetic polymorphism markers throughout the genome, and the first chromosome and the fourth chromosome (2471 markers) are analyzed first. It is noted that the inter-marker spacing for 57 % of the MS genetic polymorphism markers used for the first chromosome and 59 % used for the fourth chromosome is below 100 kb.

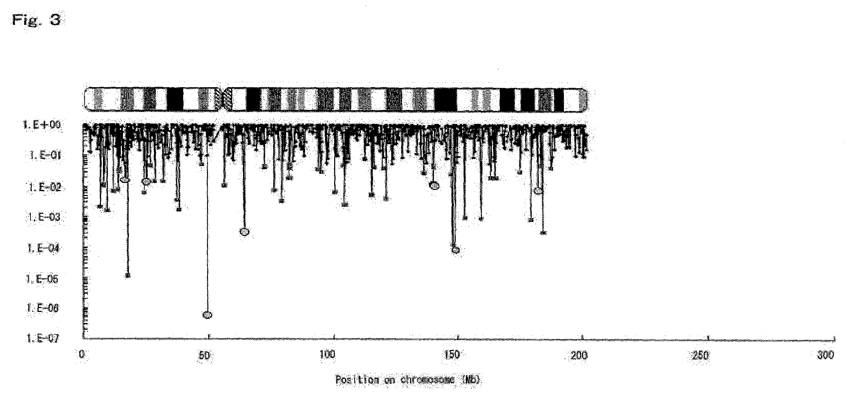

The result from performing the first screening is shown in FIG. 3. At this time, MS genetic polymorphism markers with correlation are selected through the chi-square test using a 2 x m divided table and the Fisher's exact test using a 2 x 2 divided table. Comparison of the results from using 2xm and 2x2 divided tables show a difference in the number of markers, which indicates correlation. The test using the 2x2 divided table allows detection of the difference in each allele between the patients and the healthy subjects even for the observed markers with a high allele number.

Therefore, in the case where a specific allele and susceptibility gene indicate linkage disequilibrium, the test using the 2x2 divided table allows detection. On the other hand, in the case where there are differences in the allele frequencies on the whole between the patients and the healthy subjects, the test using the 2xm divided table allows thorough detection. Considering such statistical characteristics, an MS genetic polymorphism marker is selected for performing the second screening.

In the second screening, analysis of 102 markers that have been identified as negative by the first screening is carried out. Using other DNA samples, which are different from the DNA samples used for the first screening, the pooled DNA samples to be used for the second screening are prepared and a test is carried out.

Such a step-by-step analysis is expected to allow efficient decrease in false-positive without correcting the statistic threshold. According to the report by Barcellos et al., in the case of performing the first screening using genetic polymorphism markers throughout the genome, the detected number of the MS genetic polymorphism markers found positive is expected to be the total of 1500 false-positive markers (5 % of all markers) and some of the true-positive markers. Performing the second screening In the same manner allows restriction of the number of the false-positives to 75 (5 % of 1500). Determination due to Individual typing and/ or TDT analysis or the like using other patient samples allow further decrease in the false-positives, thereby approaching identification of the susceptibility gene.

Analysis of 10 for the second screening showed 16 positive markers. Cornelis (1998) has reported about the linkage analysis of the first chromosome carried out for 114 paired incidence European Caucasians (20). They have identified significant linkage regions in D1S228 (1p36-pter)..Furthermore, Shiozawa at el. (1998) shows a significant linkage in 1p36 with a maximal lod score (MLS) being 3 or greater as the result from performing incidence pair analysis of 41 Japanese families (21). Furthermore, Jawaheer at el. (2001) shows a significant linkage in D1S235 (1q43-44) as the result from performing incidence pair analysis of 257 families from the North American Rheumatoid Arthritis Consortium (22). On the other hand, Jawaheer at el. (2001) shows a significant linkage in D4S1647 (4q23-24) of the fourth chromosome (22). According to our results provided this time, positive MS genetic polymorphism markers are observed in the reported band regions. (Example 5) Analysis of susceptibility segment for diseases scattering in entire chromosome for rheumatoid arthritis.

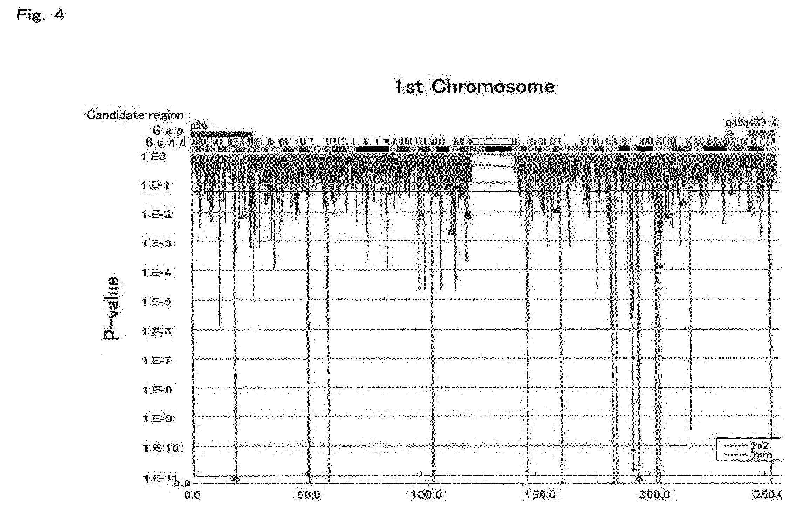

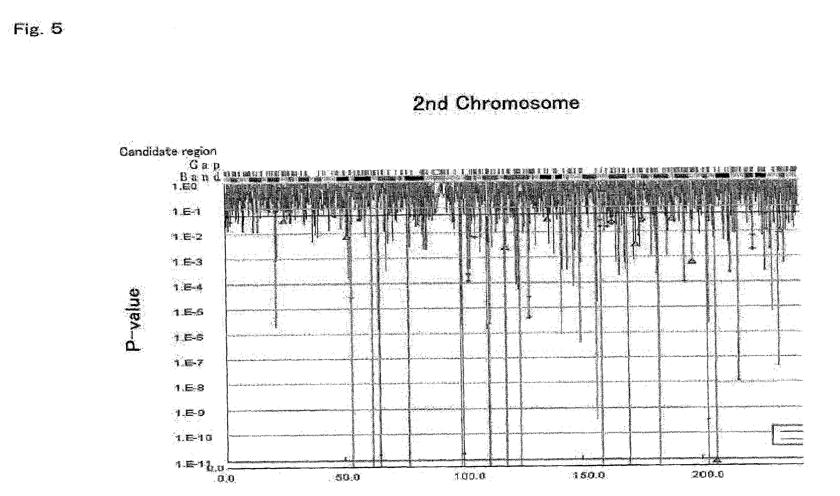

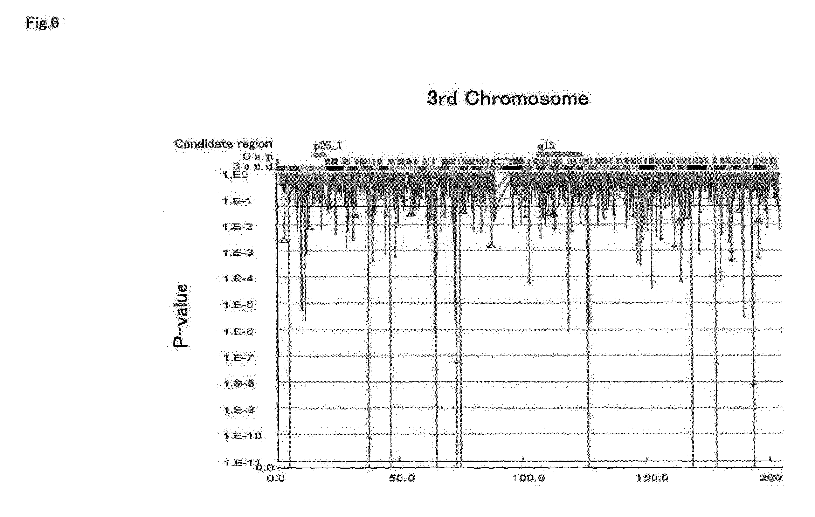

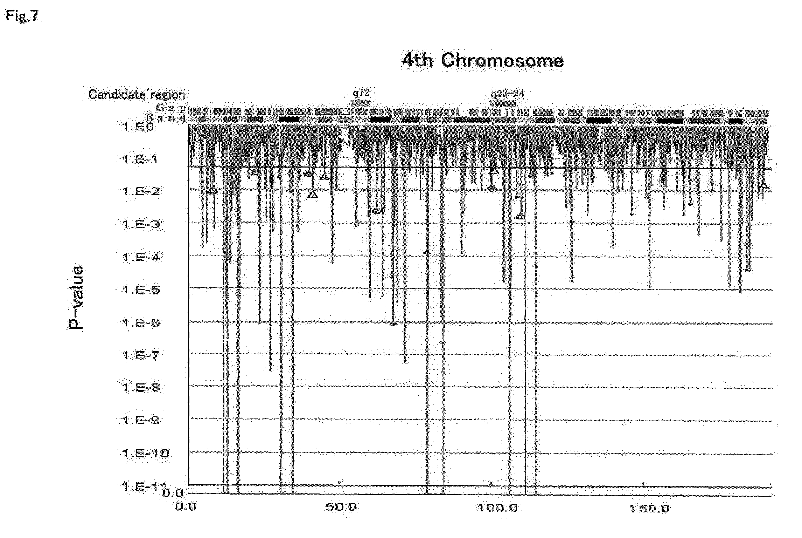

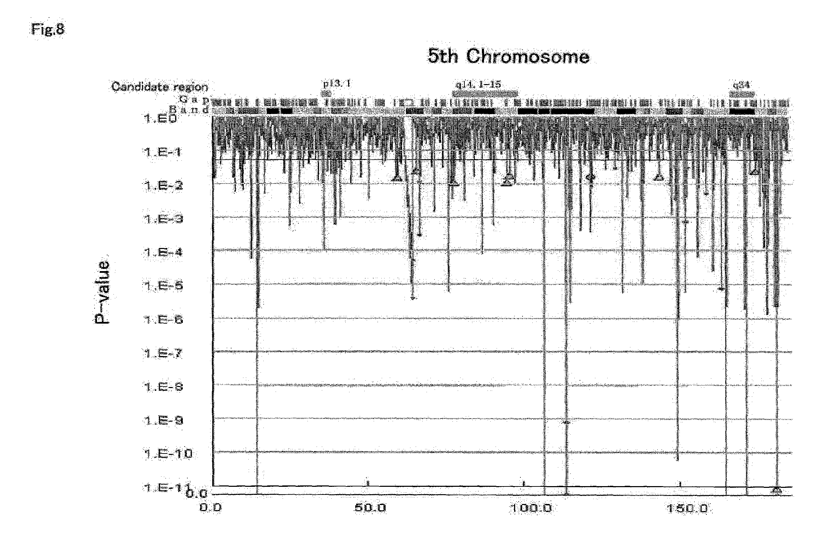

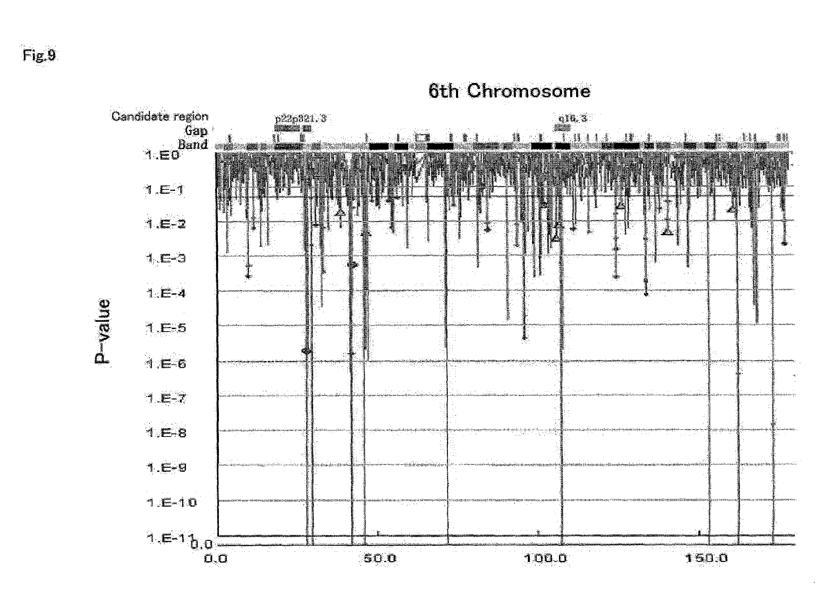

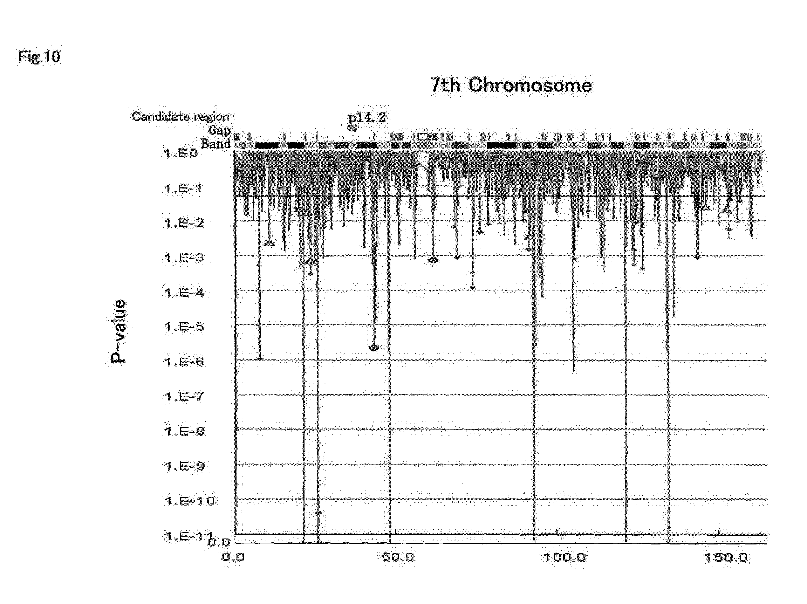

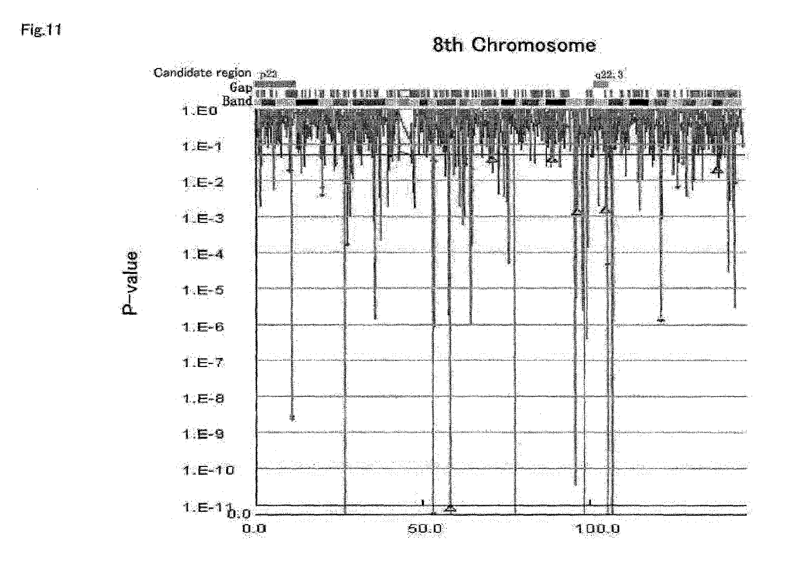

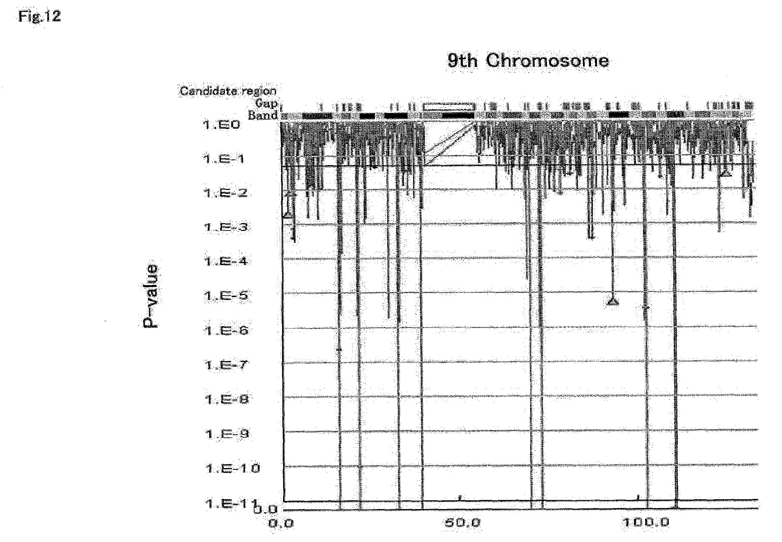

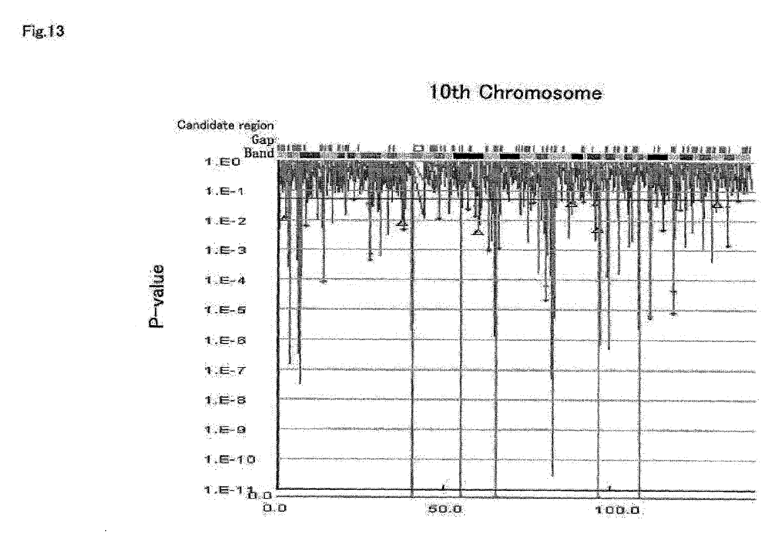

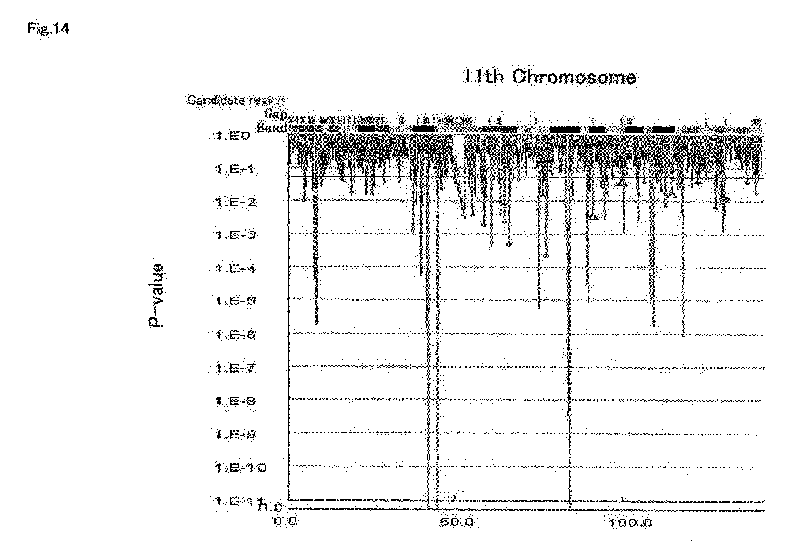

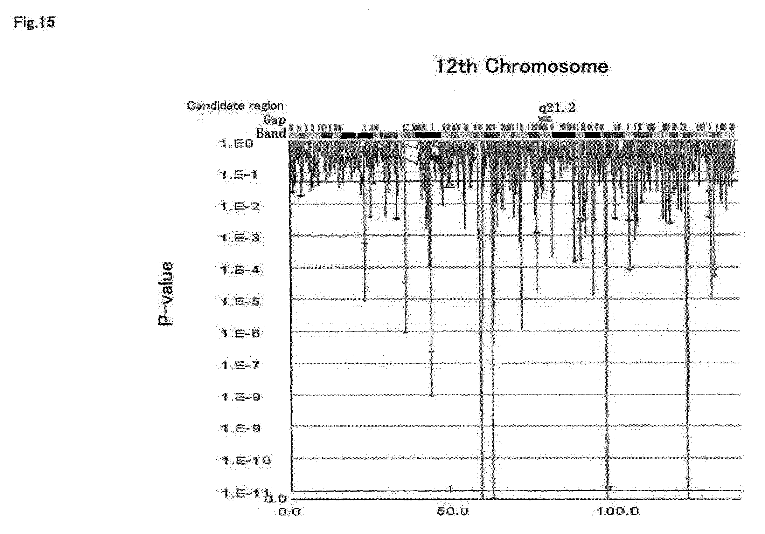

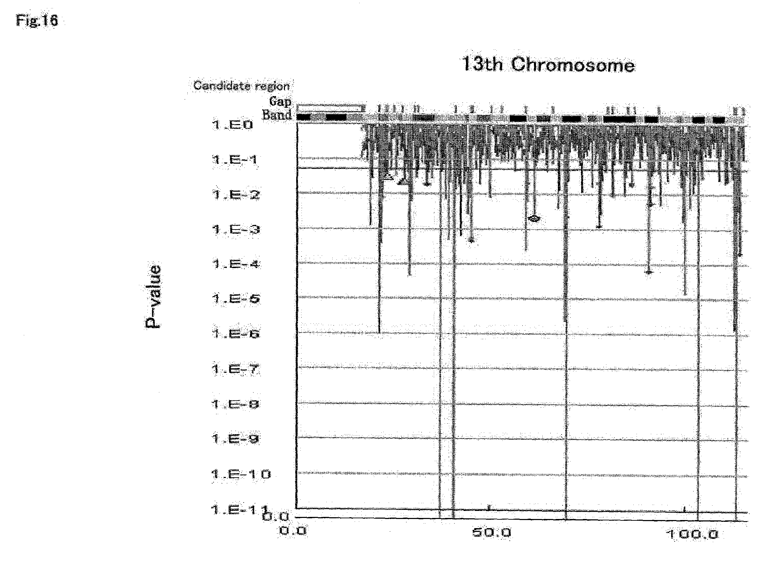

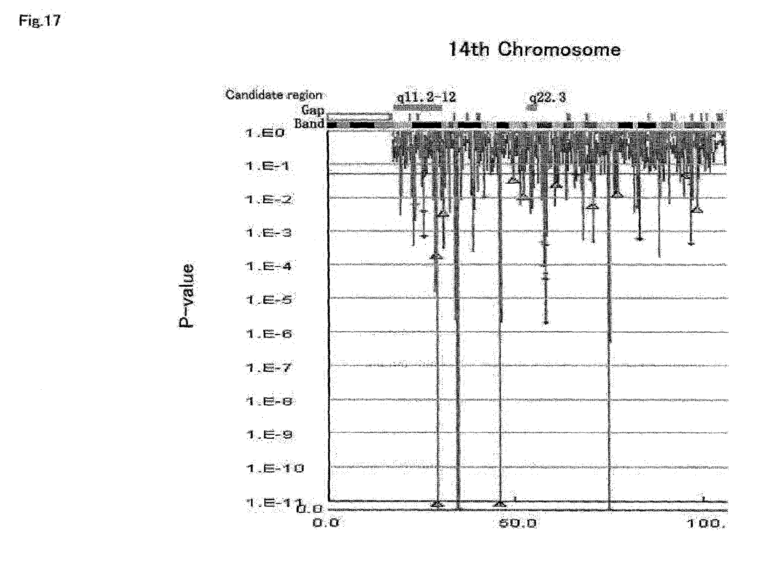

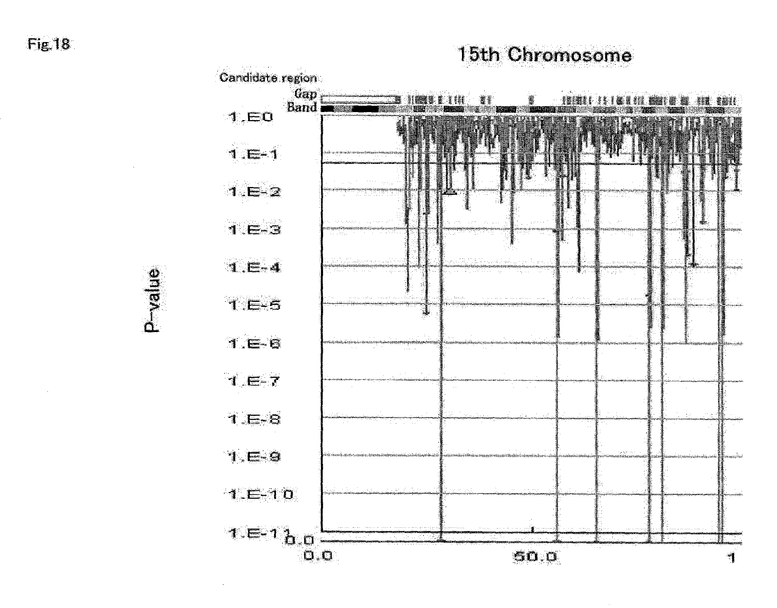

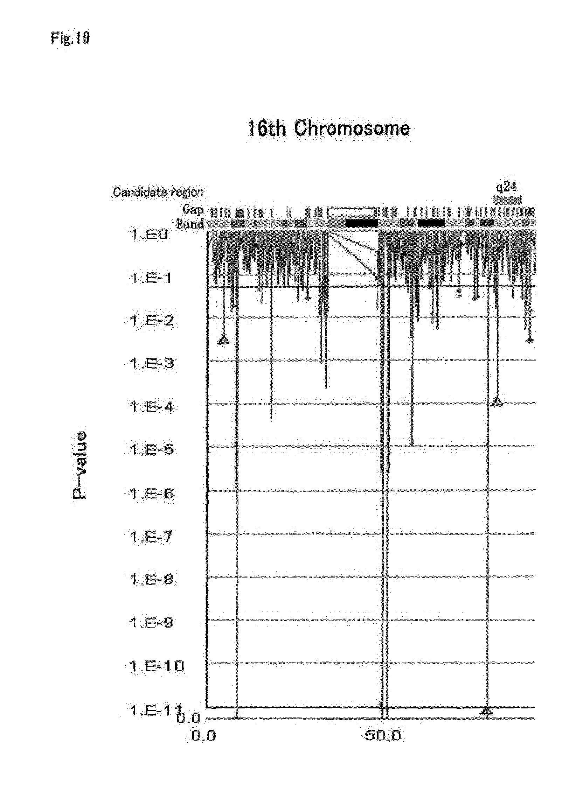

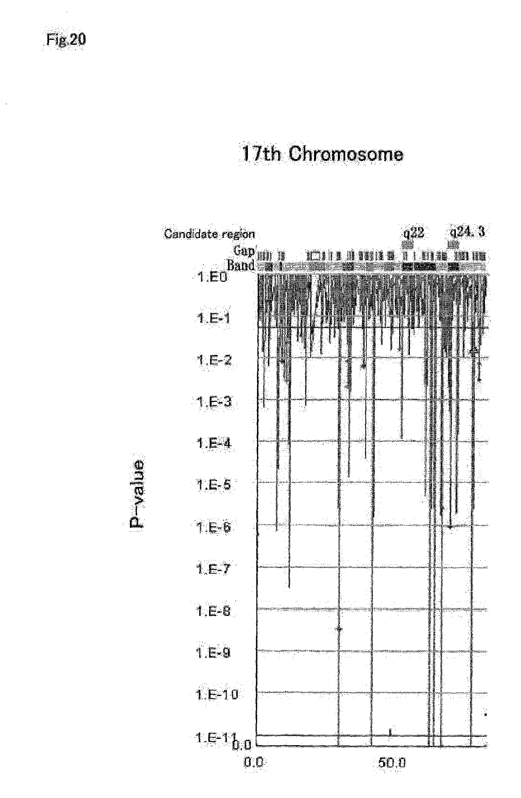

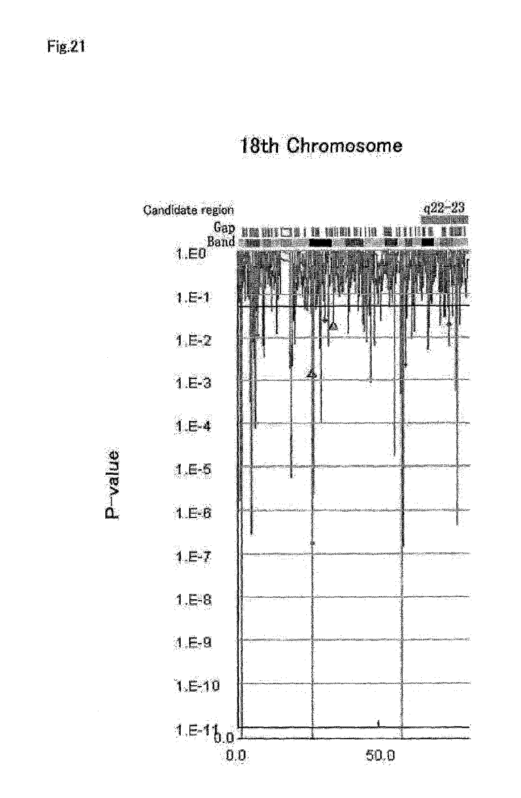

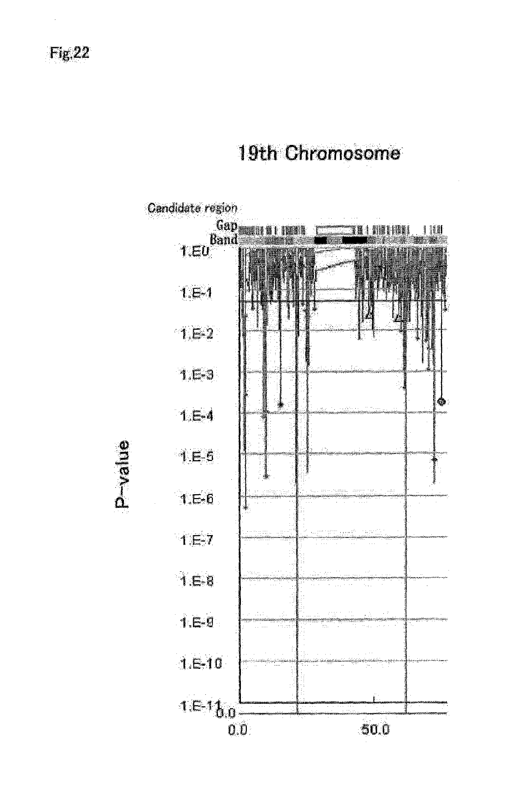

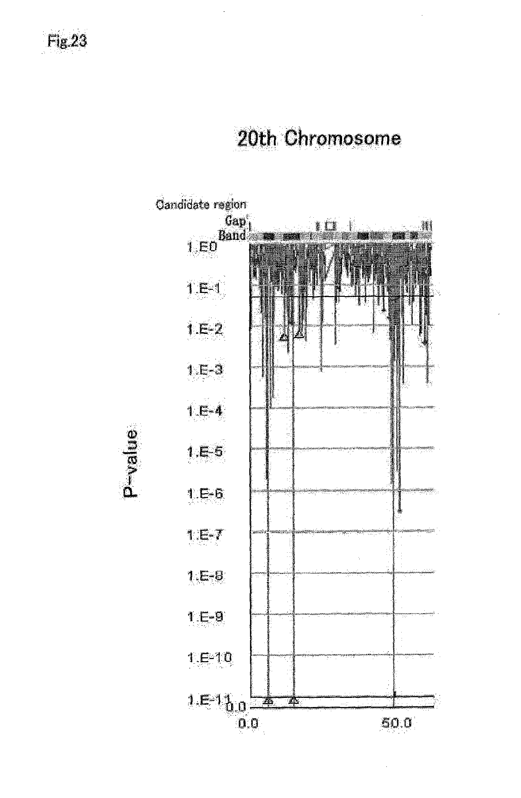

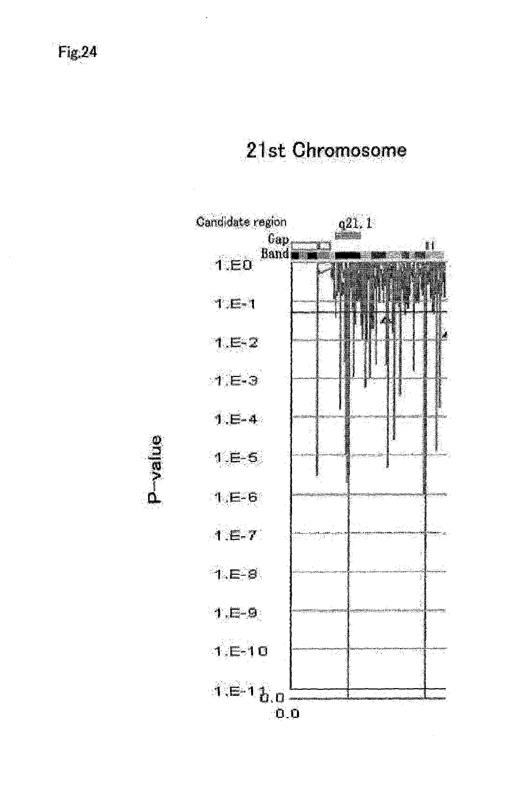

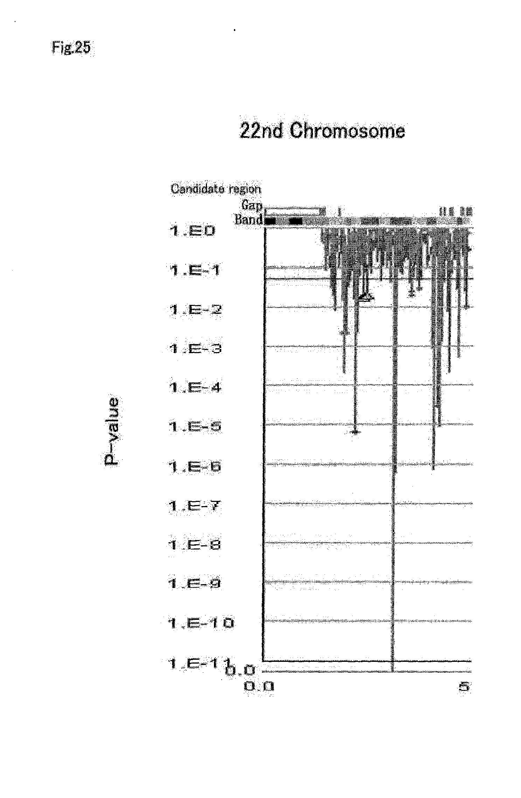

Using the samples and method used in Embodiment 4, the first and the second screening with 22,636 polymorphism microsatellites distributed throughout the chromosome as markers are performed. Moreover, the candidate segments restricted by the first and the second screening using samples of the 125 additional healthy subjects and samples of 125 additional patients for the third screening are subjected to an analysis throughout the genome or gene statistic analysis for individuals. As a result, as shown in FIGs. 4 to 25, a profile for the rheumatism susceptibility segment is identified in the first to the twenty-second chromosome except for the Y chromosome, and the X chromosome. These results are compared in quantity with the known candidate segments, and shown in Tables 4 to 6. As shown in Tables 4 to 6, three regions are known, and with a method herein, 22 regions are identified in the third screening step. Specifically, 19 additional candidate segments are identified.

Table 4

Correlation of known candidate segment with positiveness rate found by the present analysis

| | First screening | | |
| --- | --- | --- | --- |
| | No. of markers for analysis | No. of positive markers | No. of positive markers/1Mb |
| Entire chromosome* | 22,636 | 2,504 | 0.811 |
| Known candidate segment | 1,843 | 160 | 0.664 |
| Other than candidate segment | 19,432 | 2,166 | 0.761 |
| * Y chromosome is removed. | | | |

Table 5

Correlation of known candidate segment with positiveness rate found by the present analysis

| | Second screening | | |
| --- | --- | --- | --- |
| | No. of markers for analysis | No. of positive markers | No. of positive markers/1Mb |
| Entire chromosome* | 2,204 | 117 | 0.038 |
| Known candidate segment | 145 | 30 | 0.125 |
| Other than candidate segment | 1,910 | 59 | 0.021 |
| * Y chromosome is removed. | | | |

Table 6

Correlation of known candidate segment with positiveness rate found by the present analysis

| | Third screening | | |
| --- | --- | --- | --- |
| | No. of markers for analysis | No. of positive markers | No. of positive markers/1Mb |
| Entire chromosome* | 53 | 22 | 0.007 |
| Known candidate segment | 8 | 3 | 0.012 |
| Other than candidate segment | 45 | 18 | 0.006 |
| * Y chromosome is removed. | | | |

(Effect of the invention)

The method according to the present invention allows effective genetic correlation analysis throughout a genome irrelevant to race since DNA sequences including microsatellite genetic polymorphism markers prepared (sequence number 1 to sequence number 27088) have genetic polymorphism, where 95 % or greater of them are shared by all races. Analysis of 2471 markers of the first and the fourth chromosome for, for example, the rheumatoid arthritis is carried out, and since the first screening results in finding 102 markers positive, the second screening is then carried out. As a result, 16 markers are found positive (i.e., 6 markers in the first chromosome and 10 markers in the fourth chromosome). Since candidate segments in the susceptibility gene are identified, these regions are subjected to SNP analysis, which allows identification of the susceptibility gene. Moreover, in addition to usage of the microsatellite genetic polymorphism markers included DNA sequences throughout the genome (sequence number 1 to sequence number 27088), detailed analysis of only a specific chromosome for a phenotype of a disease or the like is carried out using the sequence numbers 1 to 27088, which allows higher speed and further accurate identification of the genes existing in the susceptibility segment for a disease.

Genes isolated by the method of this invention, proteins encoded by these genes, antibodies against these proteins, and/or polynucleotides containing at least 15 nucleotides complementary to one of the strands of these genes or to their complementary strands may be used for genetic screening and gene therapy. Furthermore, a pathogenic gene of a disease isolated by the method of this invention, a protein encoded by the gene, an antibody against the protein and/or a polynucleotide containing at least 15 nucleotides complementary to one of the strands of this gene or to its complementary strand may be used for testing, preventing, and/or treating the disease.

**Claims**

1.  A gene mapping method, comprising:

    collecting a DNA sample from to-be-screened subjects and control subjects;
    using forward primers having a length of 15 to 100 nucleotides and having the same nucleotide sequence as the sequence extending in 3'-direction from the 5'-terminus of each of the DNA sequences referenced with SEQ ID No: 1 to 27088 and reverse primers having a length of 15 to 100 nucleotides and having a nucleotide sequence complementary to the sequence extending in 5'-direction from the 3'-terminus of each of the DNA sequences referenced with SEQ ID No: 1 to 27088 to develop a polymerase chain reaction on the obtained DNA sample, and providing DNA sequence fragments including microsatellite genetic polymorphism markers; and
    statistically comparing the DNA sequence fragments obtained from the to-be-screened subjects with those obtained from the control subjects so as to identify an existing region of a pathogenic gene or a gene relating to human phenotypes with genetic factors.

2.  The method according to claim 1, further comprising the step of mixing the DNA samples obtained from each group into pooled DNA samples before developing the polymerase chain reaction so as to create pooled DNA samples.

3.  The method according to either claim 1 or claim 2, wherein the DNA sequences including microsatellite genetic polymorphism markers prepared are all or a part of DNA sequences referenced with SEQ ID No: 1 to 27088.

4.  The method according to either claim 1 or claim 2, wherein analysis of the microsatellite genetic polymorphism markers is carried out using a DNA chip and a mass spectrometer.

5.  A gene mapping method according to claim 1 or 2, further comprising performing a second screening of the DNA sequences including the microsatellite genetic polymorphism markers that are found positive through the method according to either claim 1 or claim 2 using DNA samples obtained from the differing to-be-screened subjects and the control subjects.

6.  A gene mapping method according to claim 1 or 2, further comprising:

    performing a genetic analysis on each group of the DNA sequences including the microsatellite genetic polymorphism markers that are found positive through the method according to either claim 1 or claim 2 using DNA samples obtained from a descent group having a pathogenic gene of to-be-analyzed subjects or a gene relating to human phenotypes with genetic factors, and identifying an existing region for the DNA sequences including the microsatellite genetic polymorphism markers that are found true-positive.

7.  A gene mapping method according to any one of claims 1, 2, 5 or 6, further comprising analyzing an existing candidate segment including a pathogenic gene or a gene relating to human phenotypes with genetic factors, which is identified using the method according to claims 1, 2, 5, or 6, based on single nucleotide polymorphisms.

8.  A set of forward primers, which have a length of 15 to 100 nucleotides and having the same nucleotide sequence as the sequences extending in 3'-direction from the 5'-terminus of each of the nucleotide sequences referenced with SEQ ID No: 1 to 27088.

9.  A set of reverse primers, which have a length of 15 to 100 nucleotides and having a nucleotide sequence complementary to the sequence extending in 5'-direction from the 3'-terminus of each of the nucleotide sequences referenced with SEQ ID No: 1 to 27088.

Fig. 1

EP 2 019 148 A1

Fig.2

Position on chromosome (Mb)

■ Correlation through first screening marker that indicates P<0.05
  but does not indicate correlation through second screening
◎ Correlation through first and second screening marker that indicates P<0.05

Fig. 3

Fig. 4

1st Chromosome

2nd Chromosome

Fig. 5

Fig.6

EP 2 019 148 A1

Fig.7

4th Chromosome

Fig.8

EP 2 019 148 A1

5th Chromosome

EP 2 019 148 A1

Fig.9

6th Chromosome

Fig.10

## 7th Chromosome

EP 2 019 148 A1

Fig.11

Fig.12

Fig.13

Fig.14

11th Chromosome

EP 2 019 148 A1

Fig.15

# 12th Chromosome

EP 2 019 148 A1

Fig.16

EP 2 019 148 A1

Fig.17

Fig.18

15th Chromosome

Fig.19

16th Chromosome

Fig.20

17th Chromosome

Fig.21

18th Chromosome

Fig.22

## 19th Chromosome

Fig.23

20th Chromosome

Fig.24

Fig.25

22nd Chromosome

Fig.26

EP 2 019 148 A1

X Chromosome

**EUROPEAN SEARCH REPORT**

Application Number

EP 08 00 6655

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | COX T C ET AL: "A very high density microsatellite map (1 STR/41 kb) of 1.7 Mb on Xp22 spanning the microphthalmia with linear skin defects (MLS) syndrome critical region."<br>EUROPEAN JOURNAL OF HUMAN GENETICS: EJHG. ENGLAND 1998 JUL-AUG,<br>vol. 6, no. 4, July 1998 (1998-07), pages 406-412, XP001179131<br>ISSN: 1018-4813<br>* the whole document *<br>----- | 1-9 | INV.<br>C12Q1/68 |
| A | VAN HEEL D A ET AL: "Identification of novel polymorphisms in the beta7 integrin gene: family-based association studies in inflammatory bowel disease."<br>GENES AND IMMUNITY. ENGLAND DEC 2001,<br>vol. 2, no. 8, December 2001 (2001-12), pages 455-460, XP009025048<br>ISSN: 1466-4879<br>* the whole document *<br>----- | 1 | |
| A | IMMERVOLL THOMAS ET AL: "Fine mapping and single nucleotide polymorphism association results of candidate genes for asthma and related phenotypes"<br>HUMAN MUTATION,<br>vol. 18, no. 4, 2001, pages 327-336, XP008014899<br>ISSN: 1059-7794<br>* the whole document *<br>-----<br><br>-/-- | 1 | **TECHNICAL FIELDS SEARCHED (IPC)**<br><br>C12Q |

The present search report has been drawn up for all claims

| Place of search | Date of completion of the search | Examiner |
|---|---|---|
| Munich | 12 December 2008 | Luzzatto, Enrico |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
........................................................................
& : member of the same patent family, corresponding
document

EPO FORM 1503 03.82 (P04C01)

**EUROPEAN SEARCH REPORT**

Europäisches Patentamt
European Patent Office
Office européen des brevets

Application Number

EP 08 00 6655

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | BARCELLOS L F ET AL: "ASSOCIATION MAPPING OF DISEASE LOCI, BY USE OF A POOLED DNA GENOMIC SCREEN" AMERICAN JOURNAL OF HUMAN GENETICS, UNIVERSITY OF CHICAGO PRESS, CHICAGO,, US, vol. 61, no. 3, 1997, pages 734-747, XP009007918 ISSN: 0002-9297 * the whole document * | 1-9 | |
| A | YOSHIKAWA Y ET AL: "AN EFFICIENT APPLICATION OF MALDI-TOF/MS COUPLED WITH MICORARRAY FOR DETECTION OF MICROSATELLITE POLYMORPHISM" AMERICAN JOURNAL OF HUMAN GENETICS, AMERICAN SOCIETY OF HUMAN GENETICS, CHICAGO, IL, US, vol. 69, no. 4, October 2001 (2001-10), page 464, XP008013150 ISSN: 0002-9297 * abstract * | 1 | |
| A | SHIOZAWA SHUNICHI ET AL: "Identification of the gene loci that predispose to rheumatoid arthritis" INTERNATIONAL IMMUNOLOGY, vol. 10, no. 12, December 1998 (1998-12), pages 1891-1895, XP002929080 ISSN: 0953-8178 * the whole document * | 1-9 | TECHNICAL FIELDS SEARCHED (IPC) |
| A | US 5 683 880 A (KAMB ALEXANDER) 4 November 1997 (1997-11-04) * the whole document * | 1-9 | |

-/--

The present search report has been drawn up for all claims

| Place of search | Date of completion of the search | Examiner |
|---|---|---|
| Munich | 12 December 2008 | Luzzatto, Enrico |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

**Application Number**

EP 08 00 6655

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 97/31011 A (YUZBASIYAN GURKAN VILMA ;UNIV MICHIGAN (US); BREWER GEORGE J (US);) 28 August 1997 (1997-08-28) * page 9, line 23 - page 29, line 12 * | 1 | |
| A | GYAPAY G ET AL: "The 1993-94 Généthon human genetic linkage map." NATURE GENETICS. UNITED STATES JUN 1994, vol. 7, no. 2 Spec No, June 1994 (1994-06), pages 246-339, XP000560918 ISSN: 1061-4036 * the whole document * | 1 | |
| A | DATABASE NCBI [Online] Homo sapiens clone CxM18 MLs syndrome 5 January 1999 (1999-01-05), COX T. C.: "Development of a very high density microsatellite map (1STR/41kb) in Xp22 reveals two exceptional non-deletion cases of microphtalmia with linear skin defects (MLS) syndrome" XP002269114 Database accession no. AF003650 * abstract * | 1-9 | |

-/--

TECHNICAL FIELDS
SEARCHED (IPC)

The present search report has been drawn up for all claims

| Place of search | Date of completion of the search | Examiner |
|---|---|---|
| Munich | 12 December 2008 | Luzzatto, Enrico |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

**EUROPEAN SEARCH REPORT**

Application Number

EP 08 00 6655

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SHIINA T ET AL: "Molecular dynamics of MHC genesis unraveled by sequence analysis of the 1,796,938-bp HLA class I region." PROCEEDINGS OF THE NATIONAL ACADEMY OF SCIENCES OF THE UNITED STATES OF AMERICA. UNITED STATES 9 NOV 1999, vol. 96, no. 23, 9 November 1999 (1999-11-09), pages 13282-13287, XP002269113 ISSN: 0027-8424 * abstract * * page 13282, column 2, line 48 - line 61 * ----- | 1 | |
| T | HIROTA Y ET AL: "Genome-wide microsatellite association studies for Parkinson's disease by using the pooled DNA method." AMERICAN JOURNAL OF HUMAN GENETICS, vol. 73, no. 5, November 2003 (2003-11), page 469, XP009024593 53rd Annual Meeting of the American Society of Human Genetics;Los Angeles, CA, USA; November 04-08, 2003 ISSN: 0002-9297 * abstract * ----- | 1,2 | TECHNICAL FIELDS SEARCHED (IPC) |
| A | WO 02/50307 A (INOKO HIDETOSHI ;KIMURA NAOKI (JP); MORIKAWA MINORU (JP); NAKAJIMA) 27 June 2002 (2002-06-27) -& EP 1 350 851 A (CHUGAI SEIYAKU K K) 8 October 2003 (2003-10-08) * the whole document * ----- | 1 | |

The present search report has been drawn up for all claims

| Place of search | Date of completion of the search | Examiner |
|---|---|---|
| Munich | 12 December 2008 | Luzzatto, Enrico |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT**
**ON EUROPEAN PATENT APPLICATION NO.**                    EP 08 00 6655

This annex lists the patent family members relating to the patent documents cited in the above-mentioned European search report.
The members are as contained in the European Patent Office EDP file on
The European Patent Office is in no way liable for these particulars which are merely given for the purpose of information.

12-12-2008

| Patent document cited in search report | | Publication date | Patent family member(s) | | Publication date |
|---|---|---|---|---|---|
| US 5683880 | A | 04-11-1997 | NONE | | |
| WO 9731011 | A | 28-08-1997 | AU | 1959797 A | 10-09-1997 |
| WO 0250307 | A | 27-06-2002 | AU | 2261402 A | 01-07-2002 |
| | | | EP | 1350851 A1 | 08-10-2003 |
| | | | US | 2004248098 A1 | 09-12-2004 |
| EP 1350851 | A | 08-10-2003 | AU | 2261402 A | 01-07-2002 |
| | | | WO | 0250307 A1 | 27-06-2002 |
| | | | US | 2004248098 A1 | 09-12-2004 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP SHO6317688 B
- WO 9203918 A
- WO 932227 A
- WO 9402602 A
- WO 9425585 A
- WO 9633735 A
- WO 9634096 A


**Non-patent literature cited in the description**

- **DIB C. et al.** *Nature,* 1966, vol. 380, 152-154
- **SHIINA T. et al.** *Proc. Natl. Acad. Sci. USA,* 1999, vol. 96, 13282-13287
- **NISHIMURA Y.** Takei no Tokeigakuteki Riyoho. *Saishin Igaku,* 1991, vol. 46, 909-923
- **OKA A. et al.** *Hum. Mol. Genetics,* 1999, vol. 8, 2165-2170
- **OTA M. et al.** *Am. J. Hum. Genet.,* 1999, vol. 64, 1406-1410
- **OZAWA A. et al.** *Tissue Antigens,* 1999, vol. 53, 263-268
- **TOMFOHRDE J. et al.** *Science,* 1994, vol. 20, 1141-1145
- **MATTHEWS, D. et al.** *Nature Genet.,* 1996, vol. 14, 231-233
- **NAIR, R.P. et al.** *Hum. Mol. Genet.,* 1997, vol. 6, 1349-1356
- **TREMBATH, R.C. et al.** *Hum. Mol Genet.,* 1997, vol. 6, 813-820
- **BRENNER W. et al.** *Arch. Dermatol. Res.,* 1978, vol. 28, 337-339
- **TIILIKAINEN A. et al.** *Br. J. Dermatol.,* 1980, vol. 102, 179-184
- **OZAWA A.** *J. Am. Acad. Dermatol.,* 1981, vol. 4, 205-230
- **CAO K. et al.** *Chin. Med. J.,* 1993, vol. 106, 132-135
- **SCHMITT-EGENOLF, M. et al.** *J. Invest. Dermatol.,* 1996, vol. 106, 711-714
- **MOLLER E. ; OLHAGEN B.** *Tissue Antigens,* 1975, vol. 6, 237-246
- **ASAHINA A. et al.** *J. Invest. Dermatol.,* 1991, vol. 97, 254-258
- **MIZUKI N. et al.** *Genomics,* 1997, vol. 42, 55-66
- **SHINA T. et al.** *Genomics,* 1998, vol. 47, 372-382
- **SHINA T. et al.** *Immunol. Rev.,* 1999, vol. 167, 193-199
- **TAKEDA J. et al.** *Nucleic Acids Res.,* 1991, vol. 20, 4613-4620
- **KRISHNAN B.R. et al.** *Genomics,* 1995, vol. 30, 53-58
- **KRISHNAN B.R. et al.** *Genomics,* 1995, vol. 30, 58-58
- **KU, D.H. et al.** *Cell Growth Differ.,* 1991, vol. 2, 179-186
- **ZHOU Y. ; CHAPLIN D.D.** *Proc. Natl. Acad. Sci. USA,* 1993, vol. 90, 9470-9474
- **ISHIHARA M. et al.** *Tissue Antigens,* 1996, vol. 48, 182-186
- **TAZI AHNINI, R. et al.** *Hum. Mol. Genet.,* 1999, vol. 8, 1135-1140
- **ALLEN M.H. et al.** *Lancet,* 1999, vol. 353, 1599-1590
- **LIU C.G. et al.** *Proc. Natl. Acad. Sci. USA,* 1996, vol. 30, 4278-4283
- **PULKKINEN L. et al.** *Hum. Mol. Genet.,* 1996, vol. 5, 1539-1546
- **BRAUN A. et al.** *Genomics,* 1997, vol. 46, 46-23
- **KRUGLYAK L.** *Nature Genetics,* 1999, vol. 17, 21-24
- **UBERBACHER E.G. ; MURAL R.J.** *Proc. Natl. Acad. Sci. USA,* 1991, vol. 88, 11261-5
- **BURGE C. ; KARLIN S.** *J. Mol. Biol.,* 1997, vol. 268, 78-94
- **SHIINA T. et al.** *Genomics,* 1998, vol. 47, 372-382
- **SHIINA, T. et al.** *Immunol. Rev.,* 1999, vol. 167, 193-199
- **SAMBROOK J. et al.** Molecular Cloning. Cold Spring Harbor Laboratory Press, 1989
- **CHIRGWIN J.M. et al.** *Biochemistry,* 1979, vol. 18, 5294-5299
- **CHOMEZYNSKI P. ; SACCHI N.** *Anal. Biochem.,* 1987, vol. 162, 156-159
- **FROHMAN M.A. et al.** *Proc. Natl. Acad. Sci. U.S.A.,* 1988, vol. 85, 8998-9002
- **BELYAVSKY A. et al.** *Nucleic Acids Res.,* 1989, vol. 17, 2919-2932

- **WARD E.S. et al.** *Nature,* 1989, vol. 341, 544-546
- **WARD E.S.** *FASEB J.,* 1992, vol. 6, 2422-2427
- **BETTER METAL.** *Science,* 1998, vol. 240, 1041-1043
- **LEI S.P. et al.** *J. Bacteriol.,* 1987, vol. 169, 4379
- *Nucleic Acids Res.,* 1990, vol. 18 (17), 5322
- **MULLIGAN R. C. et al.** *Nature,* 1979, vol. 277, 108-114
- **MIZUSHIMA S. ; NAGATA S. et al.** *Nucleic Acids Res.,* 1990, vol. 18, 5322
- **SAMBROOK J. et al.** Molecular Cloning. Cold Spring Harbor Lab. press, 1989, 5.61-5.63
- **VALLE et al.** *Nature,* 1981, vol. 291, 358-340
- **URLAUBB G. ; CHASIN L.A.** *Proc. Natl. Acad. Sci. USA,* 1980, vol. 77, 4216-4220
- **KAO F.T. ; PUCK T.T.** *Proc. Natl. Acad. Sci. USA,* 1968, vol. 60, 1275-1281
- **VICKI GLASER.** *SPECTRUM Biotechnology Applications,* 1993
- **EBERT K.M. et al.** *Bio/Technology,* 1994, vol. 12, 699-702
- **SUSUMU M. et al.** *Nature,* 1985, vol. 315, 592-594
- **MA J.K. et al.** *Eur. J. Immunol.,* 1994, vol. 24, 131-138
- Strategies for Protein Purification and Characterization: A Laboratory Course Manual. Cold Spring Harbor Laboratory Press, 1996
- **GALFRE G. ; MILSTEIN C.** *Methods Enzymol.,* 1981, vol. 73, 3-46
- **BORREBAECK C.A.K. ; LARRICK J.W.** Therapeutic Monoclonal Antibodies. MacMillan Publishers LTD, 1990
- **HUSTON J.S. et al.** *Proc. Natl. Acad. Sci. U.S.A.,* 1988, vol. 85, 5879-5883
- **CO M.S. et al.** *J. Immunol.,* 1994, vol. 152, 2968-2976
- **BETTER M. ; HORWITZ A.H.** *Methods Enzymol.,* 1989, vol. 178, 476-496
- **PLUCKTHUM A. ; SKERRA A.** *Methods Enzymol.,* 1989, vol. 178, 497-515
- **LAMOYI E.** *Methods Enzymol.,* 1986, vol. 121, 652-663
- **ROUSSEAUX J. et al.** *Methods Enzymol.,* 1986, vol. 121, 663-669
- **BIRD R.E. ; WALKER B.W.** *Trends Biotechnol.,* 1991, vol. 9, 132-137
- Antibodies: A Laboratory Manual. Cold Spring Harbor Laboratory, 1988
- **WILBUR W.J. ; LIPMAN D.J.** *Proc. Natl. Acad. Sci. USA,* 1983, vol. 80, 726-730
- Cloning and polymerase chain reaction-single-strand conformation polymorphism analysis of anonymous Alu repeats on chromosome 11. *Genomics,* 01 January 1992, vol. 12 (1), 139-146
- Detection of p53 gene mutations in human brain tumors by single-strand conformation polymorphism analysis of polymerase chain reaction products. *Oncogene,* 01 August 1991, vol. 6 (8), 1313-1318
- Multiple fluorescence-based PCR-SSCP analysis with postlabeling. *PCR Methods Appl.,* 01 April 1995, vol. 4 (5), 275-282
- **SHIINA T. et al.** *Immunol Rev.,* 1999, vol. 167, 193-199
- **TAMIYA G. et al.** *Tissue Antigens,* 1998, vol. 51, 337-346
- **TAMIYA G. et al.** *Tissue Antigens,* 1999, vol. 54, 221-228
- **GRIMALDI M. C. et al.** *Hum. Immunol.,* 1996, vol. 51, 89-94
- **WEISSENBACH J. et al.** *Nature,* 1992, vol. 29, 794-801
- **SHIINA T. et al.** *Immunol. Rev.,* 1999, vol. 167, 193-199
- **GUO S. W. ; THOMPSON E. A.** *Biometries,* 1992, vol. 48, 361-372
- **GUO S. W. ; THOMPSON E. A.** *Biometrics,* 1992, vol. 48, 361-372
- **RAYMOND M. ; ROUSSET F.** *J. Hered.,* 1995, vol. 86, 248-249
- **ROUSSET F. ; RAYMOND M.** *Genetics,* 1995, vol. 140, 1413-1419
- **JENISCH S. et al.** *Am. J. Hum. Genet.,* 1998, vol. 63, 191-199